(19) Europäisches Patentamt
European Patent Office
Office européen des brevets

(11) **EP 4 575 929 B1**

(12) **EUROPEAN PATENT SPECIFICATION**

(45) Date of publication and mention
of the grant of the patent:
**15.04.2026 Bulletin 2026/16**

(21) Application number: **24220404.8**

(22) Date of filing: **17.12.2024**

(51) International Patent Classification (IPC):
**G06N 10/60** (2022.01)

(52) Cooperative Patent Classification (CPC):
**G06N 10/60**

(54) **IMPLEMENTING DENSITY FUNCTIONAL THEORY ON QUANTUM PROCESSORS THROUGH GENERALIZED GRADIENT APPROXIMATION**

IMPLEMENTIERUNG EINER DICHTEFUNKTIONALTHEORIE AUF QUANTENPROZESSOREN DURCH VERALLGEMEINERTE GRADIENTENANNÄHERUNG

MISE EN OEUVRE D'UNE THÉORIE FONCTIONNELLE DE DENSITÉ SUR DES PROCESSEURS QUANTIQUES PAR APPROXIMATION DE GRADIENT GÉNÉRALISÉE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **21.12.2023 IN 202321087617**

(43) Date of publication of application:
**25.06.2025 Bulletin 2025/26**

(73) Proprietor: **Tata Consultancy Services Limited Maharashtra (IN)**

(72) Inventors:
• **MUKHERJEE, ANIRBAN**
400096 Mumbai, Maharashtra (IN)
• **GOPAL, ANANTHAKRISHNA**
400096 Mumbai, Maharashtra (IN)
• **BANERJEE, RITAM**
400096 Mumbai, Maharashtra (IN)

(74) Representative: **Goddar, Heinz J.**
**Boehmert & Boehmert**
**Anwaltspartnerschaft mbB**
**Pettenkoferstrasse 22**
**80336 München (DE)**

(56) References cited:
• **ROSSMANNEK MAX ET AL: "Quantum HF/DFT-embedding algorithms for electronic structure calculations: Scaling up to complex molecular systems", THE JOURNAL OF CHEMICAL PHYSICS, vol. 154, no. 11, 21 March 2021 (2021-03-21), XP093141526, ISSN: 0021-9606, DOI: 10.1063/5.0029536**
• **TAEHEE KO ET AL: "Implementation of the Density-functional Theory on Quantum Computers with Linear Scaling with respect to the Number of Atoms", ARXIV.ORG, CORNELL UNIVERSITY LIBRARY, 201 OLIN LIBRARY CORNELL UNIVERSITY ITHACA, NY 14853, 13 July 2023 (2023-07-13), XP091563102**
• **ROBERT SCHADE ET AL: "Parallel Quantum Chemistry on Noisy Intermediate-Scale Quantum Computers", ARXIV.ORG, CORNELL UNIVERSITY LIBRARY, 201 OLIN LIBRARY CORNELL UNIVERSITY ITHACA, NY 14853, 11 August 2022 (2022-08-11), XP091292209**
• **ROSSMANNEK MAX ET AL: "Quantum Embedding Method for the Simulation of Strongly Correlated Systems on Quantum Computers", JOURNAL OF PHYSICAL CHEMISTRY LETTERS, vol. 14, no. 14, 6 February 2023 (2023-02-06), US, pages 1 - 8, XP093267643, ISSN: 1948-7185, DOI: 10.1021/acs.jpclett.3c00330**

- DAS SAMBIT ET AL: "DFT-FE 1.0: A massively parallel hybrid CPU-GPU density functional theory code using finite-element discretization", COMPUTER PHYSICS COMMUNICATION., vol. 280, no. C, 21 March 2022 (2022-03-21), NL, pages 1 - 55, XP093268612, ISSN: 0010-4655, DOI: 10.1016/j.cpc.2022.108473

- RYAN PEDERSON ET AL: "Large scale quantum chemistry with Tensor Processing Units", ARXIV.ORG, CORNELL UNIVERSITY LIBRARY, 201 OLIN LIBRARY CORNELL UNIVERSITY ITHACA, NY 14853, 13 December 2022 (2022-12-13), XP091392282, DOI: 10.1021/ ACS.JCTC.2C00876

**Description**

TECHNICAL FIELD

The disclosure herein generally relates to quantum computing, and, more particularly, to implement density functional theory on quantum processors through generalized gradient approximation to extract properties of chemical compounds.

BACKGROUND

Several pharma and material science companies have successfully synthesized more than a million chemical compounds for practical use. These have been listed across various curated databases. Each molecule and its associated conformations have several quantities to be computed like solubility, stability, binding energy, electron density, atomic spectra, reaction rates, scattering cross-section, response properties and the like. The field of classical computational chemistry creates databases of these computed features. Screening drug molecules for target properties involves computational chemistry calculations. With the best of classical computational facilities 30 drug lead and its conformations in pharma each involving several hundred to few thousand atoms can be analyzed for physical properties per day with costly but accurate electronic structure approaches. It means given this large space of billions of predicted chemical compounds, a high-throughput screening job of discovering drug leads each involving several hundred to few thousand atoms currently takes about 3-5 years.

Most industrially relevant chemical systems in material sciences industry are bulk materials. One supercell made from a collection of neighboring unit cells involves around thousands of atoms which corresponds to several thousands of electronic orbitals even in the least correlated basis. The currently available techniques calculate the energetics and other physical properties of physical/ chemical systems using a quantum mechanical (QM) electronic self-consistent field approach called the Kohn-Sham Density functional theory (KS-DFT). Currently these chemical systems are treated approximately within the Quantum Mechanical (QM) or Molecular Mechanical (MM) approach, where only a subsystem with strong quantum correlations is simulated using DFT and the rest is treated only via the MM technique. Identifying these subsystems requires doing prior MM and/or force field analysis that adds to additional overhead cost. The gap in applying this DFT technology to the complete system arises from the quartic and cubic scaling wall bottleneck i.e. for a 50000 electronic orbital system (corresponding to a supercell of the bulk material), computing even one KS-DFT step would require 0.001 secs on the FUGAKU- the world's second most powerful supercomputer with a Rmax of 442 PETA Flops. For all practical purposes the DFT code should converge within 100 self-consistency steps therefore to simulate one large supercell on FUGAKU would require 0.1 seconds. For real world simulations of chemical compounds, one would require screening across several lakhs of molecular configurations in a supercell and that would require more than one day. The output electronic density computed from DFT needs to be in turn passed into Force-Field or MM modelling suites that then recomputes the geometric positioning of atoms and the DFT energies needs to be recomputed. Hence, for a bit larger system such calculations can enter several days of calculations even on the largest of the supercomputers. Similarly, the chemical systems in Pharma industry that correspond to Protein-drug or Protein-Protein systems involve more than thousands of atoms which corresponds to a several thousands of electronic orbitals. These chemical systems are treated approximately within the QM or MM approach, only a subsystem is treated with strong quantum correlations and is simulated using DFT and the rest is treated only via the MM technique. Even the drug molecules in the pharma industry have more than 500 molecular weight, therefore screening across different drug molecules enters across several days of efforts.

All the classical computational chemistry approaches rely on quantum chemistry calculations for describing the electronic density distribution profile. If that is computed at different physical conditions, then it can enable to further compute all the different physical properties. Today, most of the practical computations in pharma and material science are carried out by the Kohn Sham (KS), Density Functional Theory (DFT) and the Hartree-Fock (HF) approach. The computational complexity scales cubically to the system size which is the consequence of the delocalized nature of the wave functions which are the eigen solutions of the Kohn-Sham single particle Hamiltonian. To scale the DFT calculations to large systems, there have been attempts to develop an algorithm which scales linearly with system size. One example of such an algorithm is ONETEP (order-N electronic total energy package) linear-scaling density functional theory (DFT) calculations with large basis set (plane-wave) accuracy on parallel computers. It uses a basis of non-orthogonal generalized Wannier functions (NGWFs) expressed in terms of periodic cardinal sine (psinc) functions, which are in turn equivalent to a basis of plane-waves. ONETEP therefore is a combination of the benefits of linear scaling with a level of accuracy and variational bounds comparable to that of traditional cubic-scaling plane-wave approaches. During the calculation, the density matrix and the NGWFs are optimized with localization constraints. It is primarily designed for periodic systems, which can restrict its application to certain materials and structures. Although ONETEP is efficient for large systems, there are still practical limitations to the system size that can be treated, and the method may not be suitable for extremely large systems. Overall, ONETEP is a powerful and efficient method for performing large-scale DFT calculations in condensed matter physics and materials science. Its linear scaling and localized orbital approach make it well-suited for studying complex materials, but its application is primarily limited to periodic systems.

Currently the efforts are towards speeding up the DFT calculations using Exa scale computing: CPU, GPU, MPI, Multiprocessing etc. However, all of these have memory and processing limitations. As quantum hardware and algorithms continue to develop, various industry sectors, particularly the pharmaceutical and material design domains, are applying quantum computation paradigm to their specific problems. There have been attempts made to implement DFT on a combination of classical and quantum processors, for example, US20220012382A1 which implements DFT on a classical processor and optimizes the DFT results on a quantum processor. However, the complex calculations are still performed on a classical processor which doesn't overcome the above mentioned bottlenecks in DFT calculations. Rossmannek Max et al: "Quantum HF/DFT-embedding algorithms for electronic structure calculations: Scaling up to complex molecular systems", proposes the embedding of the quantum electronic structure calculation into a classically computed environment obtained at the Hartree-Fock (HF) or Density Functional Theory (DFT) level of theory. Achieving this by constructing an effective Hamiltonian that incorporates a mean field potential describing the action of the inactive electrons on a selected Active Space (AS). The ground state of the AS Hamiltonian is determined by means of the Variational Quantum Eigensolver (VQE) algorithm. Taehee et al: "Implementation of the Density-functional Theory on Quantum Computers with Linear Scaling with respect to the Number of Atoms", presents a quantum algorithm that has a linear scaling with respect to the number of atoms, which is much smaller than the number of electrons. The algorithm leverages the quantum singular value transformation (QSVT) to generate a quantum circuit to encode the density-matrix, and an estimation method for computing the output electron density. In addition, presents a randomized block coordinate fixed-point method to accelerate the self-consistent field calculations by reducing the number of components of the electron density that needs to be estimated. The proposed framework is accompanied by a rigorous error analysis that quantifies the function approximation error, the statistical fluctuation, and the iteration complexity. In particular, the analysis of self-consistent iterations takes into account the measurement noise from the quantum circuit. Robert Schade et al: "Parallel Quantum Chemistry on Noisy Intermediate Scale Quantum Computers", presents parallel hybrid quantum-classical algorithm for the solution of the quantum-chemical ground-state energy problem on gate-based quantum computers. This approach is based on the reduced density-matrix functional theory (RDMFT) formulation of the electronic structure problem. For that purpose, the density-matrix functional of the full system is decomposed into an indirectly coupled sum of density-matrix functionals for all its subsystems using the adaptive cluster approximation to RDMFT. The approximations involved in the decomposition and the adaptive cluster approximation itself can be systematically converged to the exact result. The solutions for the density-matrix functionals of the effective subsystems involves a constrained minimization over many-particle states that are approximated by parametrized trial states on the quantum computer similarly to the variational quantum eigensolver. The independence of the density-matrix functionals of the effective subsystems introduces a new level of parallelization and allows for the computational treatment of much larger molecules on a quantum computer with a given qubit count. In addition, proposed algorithm techniques are presented to reduce the qubit count, the number of quantum programs, as well as its depth. Rossmannek Max et al: "Quantum Embedding Method for the Simulation of Strongly Correlated Systems on Quantum Computers" employ the projection-based embedding method for combining the variational quantum eigensolver (VQE) algorithm, although not limited to, with density functional theory (DFT). The developed VQE-in-DFT method is then implemented efficiently on a real quantum device and employed for simulating the triple bond breaking process in butyronitrile. The results presented herein show that the developed method is a promising approach for simulating systems with a strongly correlated fragment on a quantum computer. Sambit Das et al: "DFT-FE 1.0: A massively parallel hybrid CPU-GPU density functional theory code using finite-element discretization", presents DFT-FE 1.0, building on DFT-FE 0.6 [Comput. Phys. Commun. 246, 106853 (2020)], to conduct fast and accurate large-scale density functional theory (DFT) calculations (reaching ~ 100,000 electrons) on both many-core CPU and hybrid CPU-GPU computing architectures. This work involves improvements in the real-space formulation-via an improved treatment of the electrostatic interactions that substantially enhances the computational efficiency-as well as high performance computing aspects, including the GPU acceleration of all the key compute kernels in DFT-FE. This demonstrates accuracy by comparing ground-state energies, ionic forces and cell stresses on a wide range of benchmark systems against those obtained from widely used DFT codes. Ryan Pederson et al: "Large scale quantum chemistry with Tensor Processing Units" demonstrates the use of Google's cloud-based Tensor Processing Units (TPUs) to accelerate and scale up conventional (cubic-scaling) density functional theory (DFT) calculations. Utilizing 512 TPU cores, accomplishes the largest such DFT computation to date, with 247848 orbitals, corresponding to a cluster of 10327 water molecules with 103270 electrons, all treated explicitly.

SUMMARY

Embodiments of the present disclosure present technological improvements as solutions to one or more of the above-mentioned technical problems recognized by the inventors in conventional systems. The invention is set out in independent claims 1, 6 and 11. Preferred aspects of the invention are set out in the dependent claims.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is a functional block diagram of a system for implementing density functional theory on quantum processors through generalized gradient approximation, in accordance with some embodiments of the present disclosure.

FIGS. 2A, 2B and 2C, collectively referred as FIG. 2 is an exemplary flow diagram illustrating a method 200 for implementing density functional theory on quantum processors through generalized gradient approximation by the system of FIG. 1, according to some embodiments of the present disclosure.

FIG. 3 illustrates an alternative representation of the flow diagram of FIG. 2, in accordance with some embodiments of the present disclosure.

FIG. 4 illustrates a quantum circuit component to compute a J matrix in the method in FIG. 2, in accordance with some embodiments of the present disclosure.

FIG. 5 illustrates a quantum circuit component to encode a collocation matrix, in accordance with some embodiments of the present disclosure.

FIG. 6 illustrates a first quantum circuit component to obtain an X-component of a gradient of the collocation matrix in accordance with some embodiments of the present disclosure.

FIG. 7 illustrates a second quantum circuit component to obtain a Y-component of the gradient of the collocation matrix in accordance with some embodiments of the present disclosure.

FIG. 8 illustrates a third quantum circuit component to obtain a Z-component of the gradient of the collocation matrix in accordance with some embodiments of the present disclosure.

FIG. 9 illustrates a quantum circuit component to encode an electronic density in accordance with some embodiments of the present disclosure.

FIG. 10 illustrates a fourth quantum circuit component to sequentially encode the collocation matrix, a density matrix, and the gradient of the collocation matrix in accordance with some embodiments of the present disclosure.

FIG. 11 illustrates a fifth quantum circuit component to sequentially encode the gradient of the collocation matrix, the density matrix, and the collocation matrix in accordance with some embodiments of the present disclosure.

FIG. 12 illustrates a quantum circuit component to obtain an electronic density gradient by composing the fourth quantum circuit component and the fifth quantum circuit component in accordance with some embodiments of the present disclosure.

FIG. 13 illustrates a quantum circuit component to encode a Z-matrix by composing a sixth quantum circuit component and the collocation matrix in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

The present disclosure provides a method for implementing density functional theory on quantum processors through generalized gradient approximation (GGA). Kohn-Sham (KS) hybrid Density Functional Theory (DFT) process is initiated from a set of $N_b$ basis functions $\mathbb{B} = \{\phi_\mu(r)\}_{\mu=1}^{N_b}$ to compute the core Hamiltonian $h$ which is a $N_b \times N_b$ matrix. $h$ constitutes the free particle energy of electrons and one-body nuclear potential arising from the electron-nucleus interactions, both these terms are independent of electronic density. Therefore h needs to be computed while initiating the self-consistent field (SCF) procedure. The direct ($J$) matrix, exact-exchange (K) matrix are electronic density dependent terms and are computed from the Electronic Repulsion Integral (ERI) tensor. The correlation exchange potential or the correlation exchange matrix ($V^{xc}$) is computed from the electronic density, its gradient and/or the Kinetic energy density. Together $J$, $K$ and $V^{xc}$ make the hybrid Kohn-Sham Fock Matrix functional F as given by equation 1.

$$\mathbf{F}[\mathbf{D}] = \mathbf{h} + \mathbf{J}[\mathbf{D}] - c\mathbf{K}[\mathbf{D}] + \mathbf{V}^{xc}[\mathbf{D}] \qquad (1)$$

In equation 1, $D$ is the one-particle density matrix discretized in the basis of $\{\phi_\mu\}$ and $c \in \mathbb{R}^+$ is the modulation for the exact exchange term. The terms $J$, $K$ can be computed from the AO-integral approach using the ERI tensor $\langle ij|kl \rangle$ and $D$ as in equations 2 and 3, respectively.

$$J_{ij} = \sum_{kl} \langle ij|kl \rangle D_{kl} \qquad (2)$$

$$K_{ij} = \sum_{kl} \langle ik|jl \rangle D_{kl} \qquad (3)$$

In equations 2 and 3, the ERI tensor $\langle ij|kl \rangle$ is obtained by integrating the space of basis functions in $\mathbb{R}^3$ according to equation 4.

$$\langle ij|kl \rangle = \int d^3 r d^3 \mathbf{r}' \phi_i(\mathbf{r}) \phi_j(\mathbf{r}) \frac{1}{|\mathbf{r}-\mathbf{r}'|} \phi_k(\mathbf{r}') \phi_l(\mathbf{r}'). \qquad (4)$$

The correlation-exchange potential $V^{xc}$ in the discretized basis $\{\phi_i\}$ is computed from equation 5, where the basis functions are defined according to equation 6.

$$V_{ij}^{xc} = \int_{R^3} d^3 \mathbf{r} \phi_i(\mathbf{r}) \frac{\delta \mathrm{E}^{xc}[\rho(\mathbf{r})]}{\delta \rho(\mathbf{r})} \phi_j(\mathbf{r}). \qquad (5)$$

$$\phi_a(\mathbf{r}) = (x - A_x)^{a_x} (y - A_y)^{a_y} (z - A_z)^{a_z} \exp(-\vartheta(x - A_x)^2) \exp\left(-\vartheta\left(y - A_y\right)^2\right) \exp(-\vartheta(z - A_z)^2) \qquad (6)$$

In equation 6, $A_x$, $A_y$, $A_z$ are nuclear coordinate locations of the Gaussian functions, $a = (a_x, a_y, a_z)$ are the integer cartesian quanta and $\vartheta$ is the cartesian Gaussian exponent. In equation 5, $\mathrm{E}^{xc}$ is the exchange energy functional evaluated for the electronic density $r$ and its form depends on the DFT method being used. The ERI tensor is represented using the Cholesky decomposition representation as given by equation 7.

$$\langle ij|kl \rangle = \sum_P L_{ij}^P L_{kl}^P \qquad (7)$$

The Cholesky matrices $L^P$ can be obtained from the Density-Fitting (DF) or the resolution of identity (RI) method as in equation 8.

$$L_{ij}^P = \frac{1}{\sqrt{w_P}} \sum_Q u_{QP} \langle ij|Q \rangle. \qquad (8)$$

In equation 8, $\langle ij|Q \rangle$ are the 3-center 2-electron integrals represented in terms of the auxiliary basis functions $\{\chi_P(\mathbf{r})\}_{P=1}^{Naux}$, and $u_{OP}$, $w_P$ are obtained from the spectral decomposition of the 2-center 2-electron integrals $V_{PQ} = \sum_R u_{PR} w_R u_{QR}^*$ represented in the auxiliary basis. The 3-center 2-electron integrals are given by equation 9 and the two center $V_{PQ} = \langle P|Q \rangle$ represents two center two electron integrals given by equation 10.

$$\langle ij|P \rangle = \int d^3 \mathbf{r} d^3 \mathbf{r}' \frac{\phi_i(\mathbf{r}) \phi_j(\mathbf{r}) \Lambda_P(\mathbf{r}')}{|\mathbf{r}-\mathbf{r}'|} \qquad (9)$$

$$\langle P|Q \rangle = \int d^3 \mathbf{r} \frac{\Lambda_P(\mathbf{r}) \Lambda_Q(\mathbf{r})}{|\mathbf{r}-\mathbf{r}'|} \qquad (10)$$

The $J$ and $K$ matrix elements are computed using the RI approach as in equation 11.

$$J_{ij} = \sum_P L_{ij}^P \sum_{kl} L_{kl}^P D_{kl}, K_{ij} = \sum_{Pl} L_{il}^P \sum_k L_{jk}^P D_{kl} \qquad (11)$$

When the KS-DFT is classically computed, the complexity of computing $J$ and $K$ is $O(pN^2)$ and this complexity resides in computing $K$. Computing h and $V^{xc}$ has lower complexity of $O(N^2)$.

Next step of DFT is to solve the generalized eigenvalue problem for the Fock matrix accounting for the overlap between basis functions $S_{ij} = \langle \phi_i | \phi_j \rangle$ and the density matrix $\boldsymbol{D}^l$ of a current iteration as given by equation 12.

$$F[\boldsymbol{D}^{(l)}]C^{(l+1)} = SC^{(l+1)}\hat{E}^{(l+1)} \qquad (12)$$

In equation 12, the one particle density matrix $\boldsymbol{D}^{(l)} = C^{(l)}WC^{(l)\dagger}$, where the occupancy matrix $W_{ij} = \theta(\mu - E_i)\delta_{ij}$ fills up $N_e/2$ lowest energy levels below the chemical potential $\mu$.

Conventional methods implement DFT simulations on classical processors such as central processing unit (CPU), graphical processing unit (GPU) etc. which is time consuming. Some state-of-the-art techniques implement DFT on a combination of classical and quantum processors. However, the complex calculations are still performed on a classical processor which does not overcome the bottlenecks in DFT calculations. To overcome these drawbacks, the present disclosure provides a method for implementing DFT through GGA using quantum processors. Initially, atomic coordinates of each atom in a chemical compound whose desired properties must be extracted is received. Electron integrals, a core Hamiltonian, and a collocation matrix is computed from the atomic coordinates. The core Hamiltonian is diagonalized to obtain a density matrix of the chemical compound which is further updated iteratively until a convergence criteria is satisfied. At each iteration, a direct matrix is computed from the density matrix, a correlation exchange matrix is computed from the direct matrix, a gradient of the collocation matrix, an electronic density, an electronic density gradient, and a derivative of the electronic density gradient, a Fock matrix is computed by adding the direct matrix and the correlation exchange matrix and the Fock matrix is diagonalized to obtain updated density matrix which is used in subsequent iteration. This is repeated until norm of a difference between the updated density matrix at a current iteration and the density matrix at a previous iteration is greater than a predefined threshold to obtain a final density matrix of the chemical compound which can be used to extract desired properties of the chemical compound.

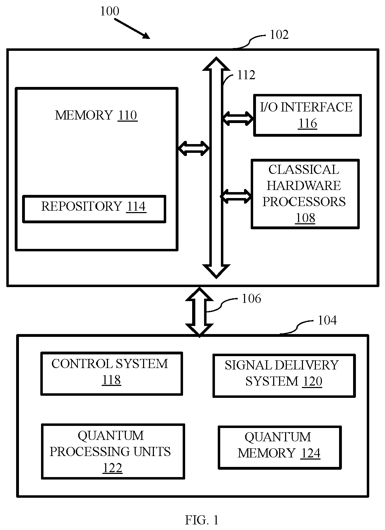

Referring now to the drawings, and more particularly to FIG. 1 through FIG. 13, where similar reference characters denote corresponding features consistently throughout the figures, there are shown preferred embodiments and these embodiments are described in the context of the following exemplary system and/or method.

FIG. 1 is a functional block diagram of a system 100 for implementing density functional theory on quantum processors through generalized gradient approximation, in accordance with some embodiments of the present disclosure. The system 100 includes a classical computing system 102, a quantum computing system 104 and a communication interface 106.

The classical computing system 102 comprises classical hardware processors 108, at least one memory such as a memory 110, an I/O interface 116. The classical hardware processors 108, the memory 110, and the Input /Output (I/O) interface 116 may be coupled by a system bus such as a system bus 112 or a similar mechanism. In an embodiment, the classical hardware processors 108 can be one or more hardware processors. The classical hardware processors and the hardware processors is interchangeably used throughout the document. Similarly, the classical computing system is a normal computing system.

The I/O interface 116 may include a variety of software and hardware interfaces, for example, a web interface, a graphical user interface, and the like., for example, interfaces for peripheral device(s), such as a keyboard, a mouse, an external memory, a printer and the like. Further, the I/O interface 116 may enable the system 100 to communicate with other devices, such as web servers, and external databases. The I/O interface 116 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 116 may include one or more ports for connecting several computing systems with one another or to another server computer.

The one or more hardware processors 108 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 108 is configured to fetch and execute computer-readable instructions stored in the memory 110.

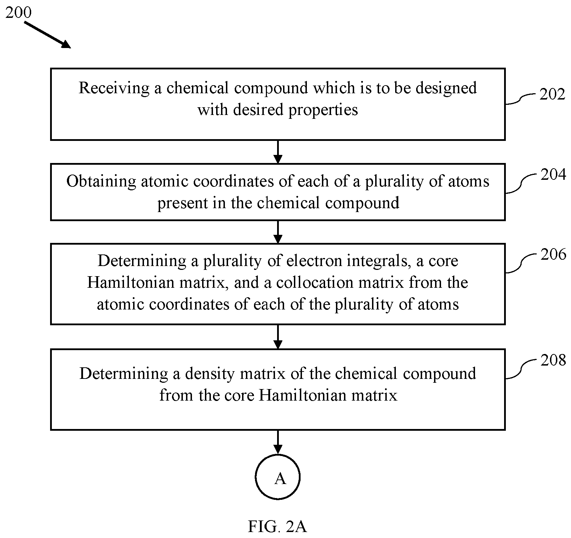

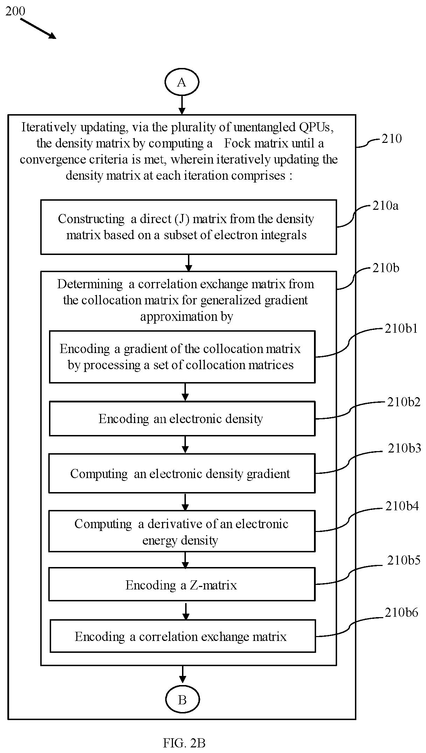

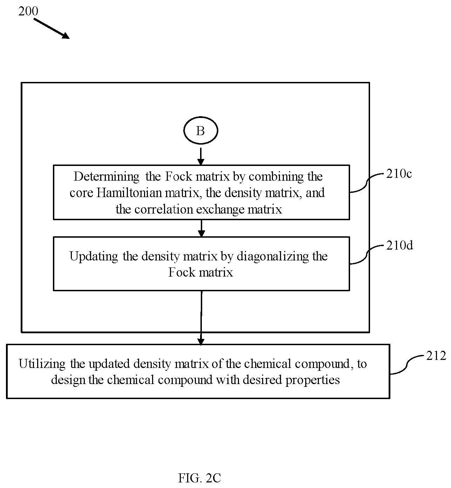

The memory 110 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. In an embodiment, the memory 110 includes a data repository 114. The data repository (or repository) 114 may include a plurality of abstracted piece of code for refinement and data that is processed, received, or generated as a result of the execution of the method illustrated in FIG.2. Although the data repository 114 is shown internal to the system 100, it should be noted that, in alternate embodiments, the data repository 114 can also be implemented external to the system 100, where the data repository 114 may be stored within a database (repository 114) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS).

The example quantum computing system 104 shown in FIG. 1 includes a control system 118, a signal delivery system 120, a plurality of Quantum Processing Units (QPUs) 122 and a quantum memory 124. The plurality of QPUs is unentangled and hence alternatively called the plurality of unentangled QPUs. The quantum computing system 104 may include additional or different features, and the components of a quantum computing system may operate as described with respect to FIG. 1 or in another manner.

The example quantum computing system 104 shown in FIG. 1 can perform quantum computational tasks (such as, for example, quantum simulations or other quantum computational tasks) by executing quantum algorithms. In some implementations, the quantum computing system 104 can perform quantum computation by storing and manipulating information within individual quantum states of a composite quantum system. For example, Qubits (i.e., Quantum bits) can be stored in and represented by an effective two-level sub-manifold of a quantum coherent physical system in the plurality of QPUs 122. In an embodiment, the quantum computing system 104 can operate using gate-based models for quantum computing. For example, the Qubits can be initialized in an initial state, and a quantum logic circuit comprised of a series of quantum logic gates can be applied to transform the qubits and extract measurements representing the output of the quantum computation. The example QPUs 122 shown in FIG.1 may be implemented, for example, as a superconducting quantum integrated circuit that includes Qubit devices. The Qubit devices may be used to store and process quantum information, for example, by operating as ancilla Qubits, data Qubits or other types of Qubits in a quantum algorithm. Coupler devices in the superconducting quantum integrated circuit may be used to perform quantum logic operations on single qubits or conditional quantum logic operations on multiple qubits. In some instances, the conditional quantum logic can be performed in a manner that allows large-scale entanglement within the QPUs 122. Control signals may be delivered to the superconducting quantum integrated circuit, for example, to manipulate the quantum states of individual Qubits and the joint states of multiple Qubits. In some instances, information can be read from the superconducting quantum integrated circuit by measuring the quantum states of the qubit devices. The QPUs 122 may be implemented using another type of physical system.

The example QPUs 122, and in some cases all or part of the signal delivery system 120, can be maintained in a controlled cryogenic environment. The environment can be provided, for example, by shielding equipment, cryogenic equipment, and other types of environmental control systems. In some examples, the components in the QPUs 122 operate in a cryogenic temperature regime and are subject to very low electromagnetic and thermal noise. For example, magnetic shielding can be used to shield the system components from stray magnetic fields, optical shielding can be used to shield the system components from optical noise, thermal shielding and cryogenic equipment can be used to maintain the system components at controlled temperature, etc.

In the example shown in FIG. 1, the signal delivery system 120 provides communication between the control system 118 and the QPUs 122. For example, the signal delivery system 120 can receive control signals from the control system 118 and deliver the control signals to the QPUs 122. In some instances, the signal delivery system 120 performs preprocessing, signal conditioning, or other operations to the control signals before delivering them to the QPUs 122. In an embodiment, the signal delivery system 120 includes connectors or other hardware elements that transfer signals between the QPUs 122 and the control system 118. For example, the connection hardware can include signal lines, signal processing hardware, filters, feedthrough devices (e.g., light-tight feedthroughs, etc.), and other types of components. In some implementations, the connection hardware can span multiple different temperature and noise regimes. For example, the connection hardware can include a series of temperature stages that decrease between a higher temperature regime (e.g., at the control system 118) and a lower temperature regime (e.g., at the QPUs 122).

In the example quantum computer system 104 shown in FIG. 1, the control system 118 controls operation of the QPUs 122. The example control system 118 may include data processors, signal generators, interface components and other types of systems or subsystems. Components of the example control system 118 may operate in a room temperature regime, an intermediate temperature regime, or both. For example, the control system 118 can be configured to operate at much higher temperatures and be subject to much higher levels of noise than are present in the environment of the QPUs 122. In some embodiments, the control system 118 includes a classical computing system that executes software to compile instructions for the QPUs 122. For example, the control system 118 may decompose a quantum logic circuit or quantum computing program into discrete control operations or sets of control operations that can be executed by the hardware in the QPUs 122. In some examples, the control system 118 applies a quantum logic circuit by generating signals that cause the Qubit devices and other devices in the QPUs 122 to execute operations. For instance, the operations may correspond to single-Qubit gates, two-Qubit gates, Qubit measurements, etc. The control system 118 can generate control signals that are communicated to the QPUs 122 by the signal delivery system 120, and the devices in the QPUs 122 can execute the operations in response to the control signals.

In some other embodiments, the control system 118 includes one or more classical computers or classical computing components that produce a control sequence, for instance, based on a quantum computer program to be executed. For example, a classical processor may convert a quantum computer program to an instruction set for the native gate set or architecture of the QPUs 122. In some cases, the control system 118 includes a microwave signal source (e.g., an arbitrary waveform generator), a bias signal source (e.g., a direct current source) and other components that generate control signals to be delivered to the QPUs 122. The control signals may be generated based on a control sequence provided, for instance, by a classical processor in the control system 118. The example control system 118 may include conversion hardware that digitizes response signals received from the QPUs 122. The digitized response signals may be provided, for example, to a classical processor in the control system 118.

In some embodiments, the quantum computer system 104 includes multiple quantum information processors that operate as respective quantum processor units (QPU). In some cases, each QPU can operate independent of the others. For instance, the quantum computer system 104 may be configured to operate according to a distributed quantum computation model, or the quantum computer system 104 may utilize multiple QPUs in another manner. In some implementations, the quantum computer system 104 includes multiple control systems, and each QPU may be controlled by a dedicated control system. In some implementations, a single control system can control multiple QPUs; for instance, the control system 118 may include multiple domains that each control a respective QPU. In some instances, the quantum computing system 104 uses multiple QPUs to execute multiple unentangled quantum computations (e.g., multiple Variational Quantum Eigen solver (VQE)) that collectively simulate a single quantum mechanical system.

In an embodiment, the quantum memory 124 is a quantum-mechanical version of classical computer memory. The classical computer memory stores information such as binary states and the quantum memory 124 stores a quantum state for later retrieval. These states hold useful computational information known as Qubits. In an embodiment, the communication interface 106 which connects the classical computing system 102 and the quantum computing system 104 is a high speed digital interface.

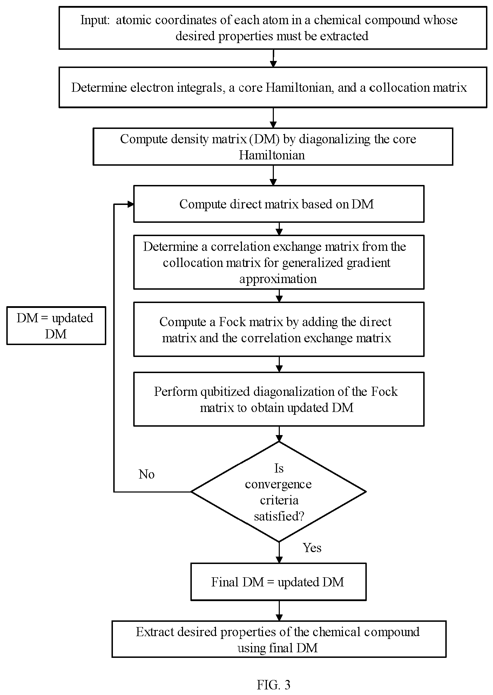

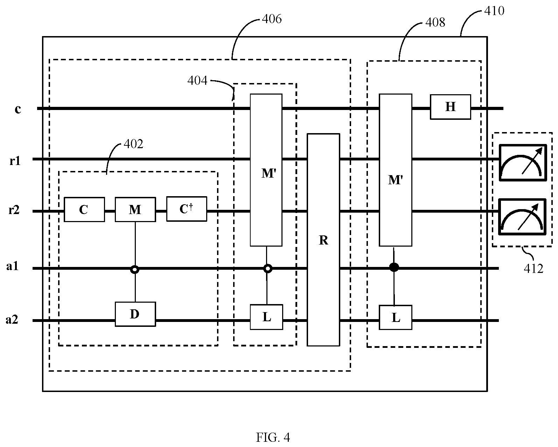

FIGS. 2A, 2B and 2C, collectively referred as FIG. 2 is an exemplary flow diagram illustrating a method 200 for implementing density functional theory through generalized gradient approximation on quantum processors by the system of FIG. 1, according to some embodiments of the present disclosure and FIG. 3 is an alternate representation of FIG. 2. In an embodiment, the system 100 includes one or more data storage devices or the memory 110 operatively coupled to the one or more hardware processor(s) 108 and is configured to store instructions for execution of steps of the method 200 by the one or more hardware processors 108. The steps of the method 200 of the present disclosure will now be explained with reference to the components or blocks of the system 100 as depicted in FIG. 1 and the steps of flow diagram as depicted in FIGS. 2 and 3, and the quantum circuits shown in FIG. 4 through FIG. 13. The method 200 may be described in the general context of computer executable instructions. Generally, computer executable instructions can include routines, programs, objects, components, data structures, procedures, modules, functions, etc., that perform particular functions or implement particular abstract data types. The method 200 may also be practiced in a distributed computing environment where functions are performed by remote processing devices that are linked through a communication network. The order in which the method 200 is described is not intended to be construed as a limitation, and any number of the described method blocks can be combined in any order to implement the method 200, or an alternative method. Furthermore, the method 200 can be implemented in any suitable hardware, software, firmware, or combination thereof.

Now referring to FIG. 2, at step 202 of the method 200, one or more classical hardware processors are configured to receive a chemical compound whose one or more properties are to be extracted. The chemical compound may be a molecule or a solid, for example, in pharma industry, the chemical compound maybe drug lead and its conformations whose physical properties must be analyzed to determine feasibility of synthesizing the drug from the drug lead. The one or more properties of drug lead include large highest occupied molecular orbital- least unoccupied molecular orbital (HOMO-LUMO) gap in solvent medium, acid-base dissociation constant (*pKa*) of a drug, lower dipole moment lesser than 10 and the like. Further, at step 204 of the method 200, the one or more classical hardware processors are configured to obtain atomic coordinates of each of a plurality of atoms present in the chemical compound. In an embodiment, the atomic coordinates for a chemical compound are given as an input as a data file or xml file. In another embodiment, atomic simulation environment (ASE), an open source package may be used for obtaining the atomic coordinates.

Further, at step 206 of the method 200, the one or more classical hardware processors are configured to determine a plurality of electron integrals, a core Hamiltonian matrix, and a collocation matrix from the atomic coordinates of each of the plurality of atoms. The plurality of electron integrals include a) 4 center 2 electron integrals, b) 3 center 2 electron integrals, c) 2 center 2 electron integrals, and d) 2 center 1 electron integrals. The electron integrals a, b, c describe the Coulomb repulsion integral that are computed in atomic orbital basis and can be represented in either spherical or cartesian coordinates. The electron integral d describes the overlap between the basis states. The plurality of electron integrals are determined using tools such as LIBCINT, Python-based Simulations of Chemistry Framework (PySCF), NorthWest computational Chemistry (NWchem) etc. The collocation matrix is a rectangular matrix of dimensions ($Ng$, $Nao$), where $Ng$ represents a number of real space grid points, and $Nao$ is a number of basis functions. It comprises a plurality of basis functions of a plurality of atomic orbitals and a plurality of points on numerical grid. Each of the plurality of basis functions is a Gaussian wave function centered around the atomic coordinates.

Further at step 208 of the method 200, a plurality of unentangled QPUs, are configured to determine e a density matrix of the chemical compound from the core Hamiltonian matrix. The density matrix is constructed using (i) a single particles unitary rotation matrix which is obtained by diagonalizing the core Hamiltonian matrix, and (ii) electron occupancies. One example way of diagonalizing the core Hamiltonian is described in patent application number 202321061415.

Further determining the density matrix, at step 210 of the method 200, the plurality of unentangled QPUs, are configured to iteratively update the density matrix by computing a Fock matrix until a convergence criteria is satisfied to obtain a final density matrix of the chemical compound. The convergence criteria is said to be satisfied when norm of a difference between the updated density matrix at a current iteration and the density matrix at a previous iteration is greater than a predefined threshold value. Steps 210a to 210d are performed at each iteration to update the density matrix. At step 210a a direct matrix (J matrix) is computed from the density matrix based on a subset of electron integrals using a quantum circuit component as illustrated in FIG. 4. The quantum circuit component for computing the J matrix is mathematically represented as: $log\ Naux + 2log\ Nao + 2$ qubits where Naux is number of auxiliary basis functions in density fitting approximation of the plurality electron integrals, and Nao is the number of basis functions. Using this quantum circuit component, initially the density matrix is encoded on $log\ Nao$ qubits in the quantum circuit to form a quantum circuit component (represented by block 402 in FIG. 4). As shown in block 402, the control is loaded on r2 and data elements of the density matrix are loaded on ancilla qubit a2. The density matrix is loaded using its Eigen decomposition representation that involves: i) $^{Nao}C_2$ Givens rotations on $log\ Nao$ qubits, ii) Then block encoding a diagonal matrix of 1's in the initial Ne/2 diagonal bocks and zeros in the rest using $2log\ Nao$ qubits, iii) Another set of $^{Nao}C_2$ Givens rotation with conjugate angles on Ne/2 diagonals, where Ne is the number of electrons. This is mathematically represented by equation 13. Here $m = log\ Naux$ is the logarithm of the number of auxilliary basis functions and $n = log\ Nao$ is the logarithm of number of basis functions.

$$O(D) = I_2^{\otimes m} \otimes \left[ \left( C \otimes I_2^{\otimes n} \otimes I_2^{a_1} \right) \left( \sum_k |k,k\rangle\langle k,k| \otimes \left( \theta(\mu - E_k)I_{a1} + (1 - \theta(\mu - E_k))Y_{a1} \right) \left( C^\dagger \otimes I_2^{\otimes n} \otimes I_2^{a_1} \right) \right] \otimes I_{a2} \quad (13)$$

The readouts from the encoding of the density matrix are obtained by equation 14.

$$\langle P, i, 0,0,0 | O(D) | P, j, 0,0,0 \rangle = \sum_{k=1}^{N_e/2} C_{ik} C_{kj}^* = D_{ij}, \text{ where } N_e = 2\sum_{k=1}^{N} \theta(\mu - E_k) \quad (14)$$

Once the density matrix is encoded a Cholesky tensor is encoded on the quantum circuit to form a first Cholesky circuit component (represented by block 404 in FIG. 4). The Cholesky tensor is obtained from one or more electron integrals among the plurality of electron integrals, particularly, 3-center 2-electron and 2-center 2-electron integrals. As shown in the block 404, the controls are encoded on the $log\ Naux + 2\ log\ Nao$ qubits and the data is loaded on the ancilla qubits. This is mathematically represented by equation 15.

$$V_L = \sum_{c=0,r_1=0,r_2=0}^{Naux-1,Nao-1} \left[ |c,r_1,r_2,0\rangle\langle c,r_1,r_2,0| \otimes I_2 + |c,r_1,r_2,1\rangle\langle c,r,1| \otimes \left( L_{r_1,r_2}^{c'} I + i\sqrt{1 - \left(L_{r_1,r_2}^{c'}\right)^2} Y \right) \right] \quad (15)$$

Further the quantum circuit component is composed with the first Cholesky circuit component and a diffusion operator to create a quantum circuit component that processes the density matrix to generate an intermediate state vector as illustrated by block 406. The diffusion operator $R$ is given by equation 16.

$$R = \sum_{kl} \left[ 2\frac{|k,+,0\rangle\langle l,+,0|}{Naux} \otimes I_2^{\otimes 2n} - I \right] \quad (16)$$

Next transpose of the Cholesky tensor is encoded on the quantum circuit to form a second Cholesky circuit component as illustrated by block 408. It is mathematically represented by equation 17.

$$V'_L = \sum_{c=0,r_1=0,r_2=0}^{Naux-1,Nao-1} \left[ |c,r_1,r_2,0\rangle\langle c,r_1,r_2,0| \otimes \left( L^{c'}_{r_1,r_2} I + \right. \right.$$

$$\left. \left. i\sqrt{1 - \left(L^{c'}_{r_1,r_2}\right)^2 Y}\right) + |c,r_1,r_2,1\rangle\langle c,r,1| \otimes I_2 \right] \qquad (17)$$

Further the quantum circuit component is composed with the second Cholesky circuit component (to form block 410) for processing the intermediate state vector to obtain a plurality of states at the second set of qubits in the quantum circuit. Sequences of bitstrings are read from the second set of qubits (by block 412) to obtain the direct matrix. This step is mathematically represented by equation 18.

$$\langle 0,i,0,0,0|H^{\otimes m}V'_L R V_L O(D)|0,j,0,1,0\rangle = \sum L^a_{ij} L^a_{kl} D_{kl} = J_{ij} \qquad (18)$$

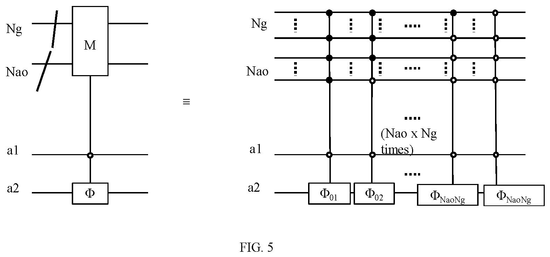

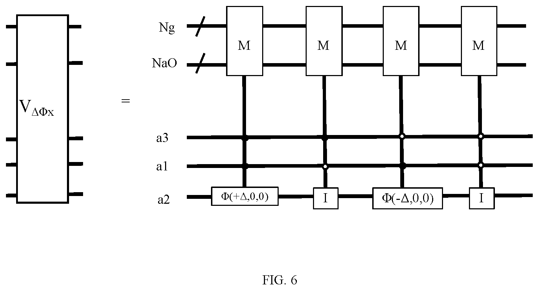

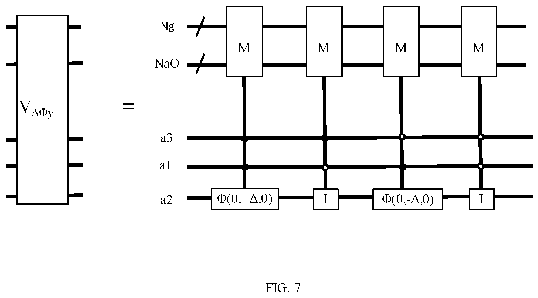

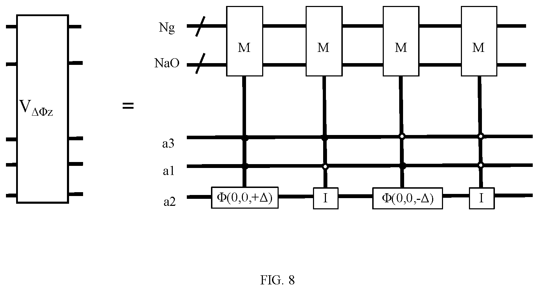

Once the direct matrix is obtained, at step 210b, a correlation exchange matrix is determined from the collocation matrix. Steps 210b1 through 210b6 provides an explanation for determining the correlation exchange matrix. The collocation matrix is encoded in a quantum circuit component as illustrated in FIG. 5, where the collocation matrix is encoded in terms of multi-qubit unitary gates in which each entry of the collocation matrix is encoded on a2 ancilla qubits with controls on NaO, Ng sets of qubits. Initially, at step 210b1, a gradient of collocation matrix is encoded by processing a set of collocation matrices. These set of collocation matrices are encoded for a set of positions (x+/-delta,y,z),(x,y+/-delta,z), (x,y,z+/-delta) for the number of basis functions at all (x,y,z) positions. The set of collocation matrices are encoded using a first quantum circuit component as shown in FIG. 6, a second quantum circuit component as shown in FIG. 7, a third quantum circuit component as shown in FIG. 8 on (a) control qubits composed of a first set of qubits and a second set of qubits, and (b) a plurality of ancilla qubits where the first set of qubits is associated with the number of points on the numerical grid, the second set of qubits is associated with the number of basis functions, and the plurality of ancilla qubits are used to store numerical entries for the set of collocation matrices. This is mathematically represented as: log Ng + log Nao + 1+2 qubits, where log Ng represents the first set of qubits, log Nao represents the second set of qubits, and 1+2 qubits represent the plurality of ancilla qubits. In FIGS. 6,7, and 8 a1, a2, and a3 represents the plurality of ancilla qubits. FIG. 6 illustrates a first quantum circuit component, where a first subset of subset of collocation matrices are encoded for a first set of grid points ((+delta x, 0,0) and (-delta x,0,0)) to obtain an X-component of the gradient of the collocation matrix. Equation 19 represents the collocation matrix,

$$\Phi = \begin{pmatrix} \phi_1(x_1,y_1,z_1), \ldots\ldots\ldots \phi_1\left(x_{N_g}, y_{N_g}, z_{N_g}\right) \\ \vdots \\ \vdots \\ \phi_{N_{ao}}(x_1,y_1,z_1), \ldots\ldots\ldots \phi_{N_{ao}}\left(x_{N_g}, y_{N_g}, z_{N_g}\right) \end{pmatrix} \qquad (19)$$

The first subset of collocation matrices are represented using equation 20,

$$\Phi_{\pm\Delta x} = \begin{pmatrix} \phi_1(x_1 \pm \Delta x,y_1,z_1), \ldots\ldots\ldots \phi_1\left(x_{N_g} \pm \Delta x, y_{N_g}, z_{N_g}\right) \\ \vdots \\ \vdots \\ \phi_{N_{ao}}(x_1 \pm \Delta x,y_1,z_1), \ldots\ldots\ldots \phi_{N_{ao}}\left(x_{N_g} \pm \Delta x, y_{N_g}, z_{N_g}\right) \end{pmatrix} (20)$$

Further FIG. 7 illustrates a second quantum circuit component, where a second subset of collocation matrices is encoded for a second set of grid points ((0, +delta y, 0) and (0, -delta y, 0)) to obtain a Y-component of the gradient of the collocation matrix. The second subset of collocation matrices are represented using equation 21,

$$\Phi_{\pm\Delta y} = \begin{pmatrix} \phi_1(x_1, y_1 \pm \Delta y, z_1), \ldots\ldots\ldots \phi_1\left(x_{N_g}, y_{N_g} + \pm\Delta y, z_{N_g}\right) \\ \vdots \\ \phi_{N_{ao}}(x_1 \pm \Delta x, y_1, z_1), \ldots\ldots\ldots \phi_{N_{ao}}\left(x_{N_g}, y_{N_g} \pm \Delta y, z_{N_g}\right) \end{pmatrix} \qquad (21)$$

Next FIG. 8 illustrates a third quantum circuit component, where a third subset of collocation matrices is encoded for a set of grid points ((0,0, +delta z) and (0,0, -delta z)) to obtain a Z-component of the gradient of the collocation matrix. The third subset of collocation matrices are represented using equation 22,

$$\Phi_{\pm\Delta z} = \begin{pmatrix} \phi_1(x_1, y_1 \pm \Delta y, z_1), \ldots\ldots\ldots \phi_1\left(x_{N_g}, y_{N_g} + \pm\Delta y, z_{N_g}\right) \\ \vdots \\ \phi_{N_{ao}}(x_1, y_1, z_1 \pm \Delta z), \ldots\ldots\ldots \phi_{N_{ao}}\left(x_{N_g}, y_{N_g} \pm \Delta y, z_{N_g} \pm \Delta z\right) \end{pmatrix} \qquad (22)$$

The X-component of the gradient of the collocation matrix is mathematically represented using equations 23.

$$V_{\partial_j\Phi} = I_2^{\otimes(nao+ng)} H I_2^{\otimes 2} H \sum_{r,c}(|c,r,0,0\rangle\langle c,r,0,0| \otimes \left[ \Phi_{+\Delta j,c,r} I + \right.$$

$$\left. i\sqrt{1 - \Phi_{+\Delta j,c,r}^2} Y \right] + |c,r,0,1\rangle\langle c,r,0,1| \otimes$$

$$I_2 + |c,r,1,0\rangle\langle c,r,1,0| \otimes \left[ \Phi_{-\Delta j,c,r} I + i\sqrt{1 - \Phi_{-\Delta j,c,r}^2} Y \right] +$$

$$|c,r,1,1\rangle\langle c,r,1,1| \otimes I_2) I_2^{\otimes(nao+ng)} H I_2^{\otimes 2} \qquad (23)$$

Similarly, $V_{\partial_y\Phi}$ and $V_{\partial_z\Phi}$ (the Y-component of the gradient of the collocation matrix and the Z-component of the gradient of the collocation matrix) are obtained using the second and third quantum circuit component. Using these a quantum circuit for $\nabla\rho$ is mathematically represented as in equations 24, 25, and 26.

$$\partial_x\rho|r_i = \langle 0,i,0,0,0|V_\Phi R_1 V_D R_1 V_{\partial_x\Phi}|0,j,0,0,0\rangle +$$

$$\langle 0,i,0,0,0|V_{\partial_x\Phi} R_1 V_D R_1 V_\Phi|0,j,0,0,0\rangle = \sum[\Phi_{ib}D_{ab}(\Phi_{\Delta X,ia} - \Phi_{-\Delta X,ia}) + (\Phi_{\Delta X,ib} -$$

$$\Phi_{-\Delta X,ib})D_{ab}\Phi_{ia}] \qquad (24)$$

$$\partial_y\rho|r_i = \langle 0,i,0,0,0|V_\Phi R_1 V_D R_1 V_{\partial_y\Phi}|0,j,0,0,0\rangle +$$

$$\langle 0,i,0,0,0|V_{\partial_y\Phi} R_1 V_D R_1 V_\Phi|0,j,0,0,0\rangle = \sum[\Phi_{ib}D_{ab}(\Phi_{\Delta Y,ia} - \Phi_{-\Delta Y,ia}) + (\Phi_{\Delta Y,ib} -$$

$$\Phi_{-\Delta Y,ib})D_{ab}\Phi_{ia}] \qquad (25)$$

$$\partial_z\rho|r_i = \langle 0,i,0,0,0|V_\Phi R_1 V_D R_1 V_{\partial_x\Phi}|0,j,0,0,0\rangle +$$

$$\langle 0,i,0,0,0|V_{\partial_x\Phi} R_1 V_D R_1 V_\Phi|0,j,0,0,0\rangle = \sum \Phi_{ib}D_{ab}(\Phi_{\Delta Z,ia} - \Phi_{-\Delta Z,ia}) + (\Phi_{\Delta Z,ib} -$$

$$\Phi_{-\Delta Z,ib})D_{ab}\Phi_{ia}] \qquad (26)$$

Then the first quantum circuit component, the second quantum circuit component, and the third quantum circuit component are composed to obtain the gradient of the collocation matrix.

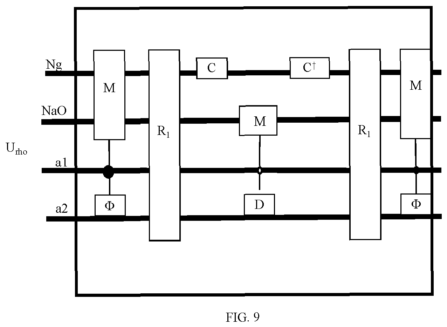

Further, at step 210b2, an electronic density is encoded by sequentially performing a matrix multiplication of the collocation matrix, the density matrix, and the collocation matrix which is illustrated in a quantum circuit component represented as $U_{rho}$. In FIG. 9, C represents unitary rotation matrix obtained from diagonalizing Fock matrix, M symbolizes the sequence of controlled unitary gates acting on the r2 that encodes the entries of the D matrix on the qubit a2, D symbolizes the occupancy matrix whose first Ne/2 entries are one and the rest is zero, C† is the Hermitian conjugate of C, and Φ =symbolizes the collocation matrix

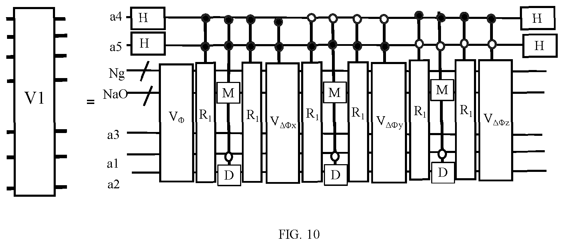

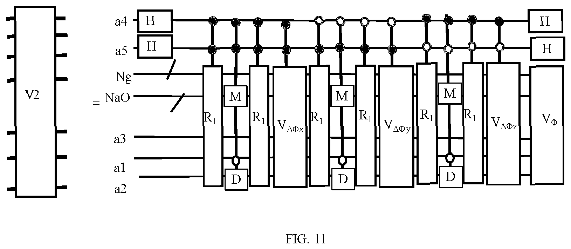

Next at step 210b3, an electronic density gradient is computed from the collocation matrix, the density matrix, and the gradient of the collocation matrix by composing a fourth quantum circuit component and a fifth quantum circuit component using at least one ancilla qubit from the plurality of ancilla qubits. The fourth quantum circuit component and a fifth quantum circuit component are shown in FIG. 10 and 11 respectively. The fourth quantum circuit component sequentially encodes the collocation matrix, the density matrix, and the gradient of the collocation matrix. The density matrix is encoded on control qubits composed of the second set of qubits and at least one ancilla qubits from the plurality of ancilla qubits. Equation 27 mathematically represents the encoding done using the fourth quantum circuit component,

$$V1 = |00\rangle\langle00| \otimes V_{\partial_x\Phi}R_1V_DR_1V_\Phi + |01\rangle\langle01| \otimes V_{\partial_y\Phi}R_1V_DR_1V_\Phi + |10\rangle\langle10| \otimes$$

$$V_{\partial_z\Phi}R_1V_DR_1V_\Phi \qquad\qquad (27)$$

The fifth quantum circuit component sequentially encodes the gradient of the collocation matrix, the density matrix, and the collocation matrix. Equation 28 mathematically represents the encoding done using the fifth quantum circuit component,

$$V2 = |00\rangle\langle00| \otimes V_{\partial_x\Phi}R_1V_DR_1V_\Phi + |01\rangle\langle01| \otimes V_{\partial_y\Phi}R_1V_DR_1V_\Phi + |10\rangle\langle10| \otimes$$

$$V_{\partial_z\Phi}R_1V_DR_1V_\Phi + |11\rangle\langle11| \otimes I \qquad\qquad (28)$$

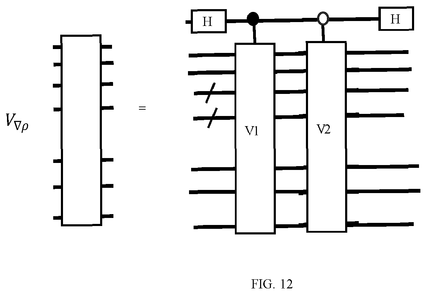

Finally, the electronic density gradient is computed by composing the fourth quantum circuit component and the fifth quantum circuit component which is shown in FIG. 12. Equation 29 mathematically represents the electronic density gradient,

$$V_{\nabla\rho} = |0\rangle\langle0| \otimes V1 + |0\rangle\langle0| \otimes V2 \qquad\qquad (29)$$

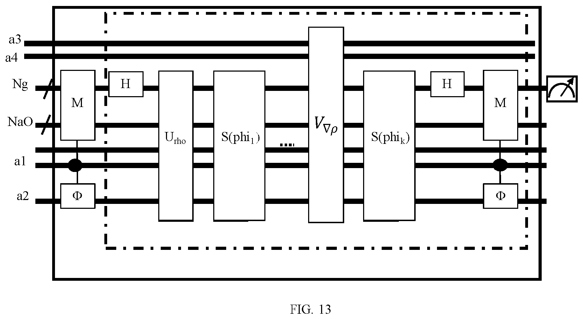

Further at step 210b4, a derivative of an electronic energy density is computed with respect to the electronic density by performing a quantum signal processing sequence on a sixth quantum circuit as shown inside the dashed line box of FIG. 13. The sixth quantum circuit component encodes the electronic density and the electronic density gradient as shown in FIG. 13. In FIG. 13, $U_{rho}$ represents the quantum circuit component for encoding the electronic density as shown in FIG. 9. The quantum signal processing is performed on the sixth quantum circuit component that encodes the electronic density and its gradient, a nonlinear function. The nonlinear function represents the derivative of the electronic energy density with respect to the electronic density. The quantum signal processing sequence is made from a sequence of phase shifted reflectors and the sixth quantum circuit component encoding the electronic density and the electronic density gradient. The phase angles for the phase shifted reflectors are obtained from the Chebyshev expansion of the nonlinear function using a classical computer. These angles can be predetermined and are determined only once. S(phi₁),...., S(phi_k) in FIG. 13 represents the phase angles.

At step 210b5 a Z-matrix is encoded by composing the sixth quantum circuit component and the collocation matrix as illustrated in FIG. 12. Equation 30 below represents the encoding of the Z-matrix,

$$V_Z = \prod_{k=1}^{N} \left[ \left(H^{\otimes nao} \otimes I^{\otimes(ng+2)}\right)V_\Phi R_1V_DR_1V_\Phi\left((1 + e^{-i\phi_k})|0,i,0,0\rangle\langle0,i,0,0| - 1\right)\right]$$

$$\prod_{k=1}^{N} \left[ \left(H^{\otimes nao} \otimes I^{\otimes(ng+2)}\right)V_{\nabla\Phi} R_1V_DR_1V_\Phi\left((1 + e^{-i\phi_k})|0,i,0,0\rangle\langle0,i,0,0| - 1\right)\right]$$

$$\left(H^{\otimes nao} \otimes I^{\otimes(ng+2)}\right)V_\Phi H^{\otimes ng} \qquad\qquad (30)$$

Finally at step 210b6, a correlation exchange matrix is encoded by composing the Z-matrix with the collocation matrix. This is represented by equation 31 below,

$$V_{ij}^{xc} = \langle i,0,0,0|H^{\otimes ng}V_{\Phi}V_Z|j,0,0,0\rangle = \sum_r \Phi_{ir}\frac{dE(\rho,\nabla\rho)}{d\rho(r)}\Phi_{jr} \qquad (31)$$

In an embodiment, theorem A is applied on equations 24 through 26 and equations 29 through 31 to implement matrix multiplications and tensor contractions on quantum circuit components with qubit count efficient resources. The theorem A states that: If $A$ and $B$ are general rectangular matrices of dimensions $dim(A) = (N, P)$ and $dim(B) = (P, M)$ then there is a unitary operation $U(A, B)$ of dimension $2^{nq} \times 2^{nq}$ that operates on a system of $n_q = p + \max(m,n) + 2$ qubit registers $|\cdot\rangle_p|\cdot\rangle_{\max}(m,n)|\cdot\rangle_{a1}|\cdot\rangle_{a2}$ (where $n = \lceil log_2 N\rceil, m = \lceil log_2 M\rceil, p = \lceil log_2 P\rceil$ )and block encodes the matrix multiplication of $A$ and $B$ such that:

$$\left\langle 0\big|_p\left\langle i\big|_{\max(m,n)}\left\langle 0\big|_{a_1}\langle 0\big|_{a_2}U(A,B)|0\rangle_p\big|j\right\rangle_{\max(m,n)}\big|1\right\rangle_{a_1}\big|0\right\rangle_{a_2} = \frac{1}{max(M,N)P}\big|\frac{\sum_k A_{ik}B_{kj}}{\|A\|\|B\|}$$

$$(32)$$

Proof of the theorem A is given below:

Take the normalized matrices $A' = A/(\sqrt{2}\|A\|)$, $B' = B/(\sqrt{2}\|B\|)$. For these define two unitary operators $V(A), V(B)$,

$$V_A = \sum_{c=0,r=0}^{2^p,2^{\max(m,n)}}[|c,r,0\rangle\langle c,r,0| \otimes \left(A'_{rc}I + i\sqrt{1-A'^2_{rc}}Y\right) + |c,r,1\rangle\langle c,r,1| \otimes I_2]$$

$$V_B = \sum_{c=0,r=0}^{2^p,2^{\max(m,n)}}[|c,r,0\rangle\langle c,r,0| \otimes I_2 + |c,r,1\rangle\langle c,r,1| \otimes \left(B'_{cr}I + i\sqrt{1-B'^2_{cr}}Y\right)]$$

$$(33)$$

The classical data of the B matrix is loaded using the state preparation oracle $V_B H^{\otimes p}$ on the initial state $|0\rangle|j\rangle|1\rangle|0\rangle$,

$$|\Phi_B\rangle = V_B H^{\otimes p}|0\rangle|j\rangle|1\rangle|0\rangle = \frac{1}{\sqrt{P}}\sum_c[B'_{cj}|c,j,1,0\rangle + \sqrt{1-B'^2_{cj}}|c,j,1,1\rangle] \qquad (34)$$

The classical data of the A matrix is loaded using the state preparation oracle $V_A H^{\otimes p}$,

$$|\Phi_A\rangle = V_A H^{\otimes p}|0\rangle|i\rangle|0\rangle|0\rangle = \frac{1}{\sqrt{P}}\sum_c[A'_{ic}|c,i,0,0\rangle + \sqrt{1-A'^2_{ic}}|c,i,0,1\rangle] \qquad (35)$$

The states $|\Phi_A\rangle$ and $|\Phi_B\rangle$ are orthogonal,

$$\langle \Phi_A|\Phi_B\rangle = 0 \qquad (36)$$

Next define diffusion operator $R$ acting on the row registers and the ancillas $a_1, a_2$,

$$R = I_2^{\otimes p} \otimes [H^{\otimes \max(m,n)} \otimes H \otimes I_2)(2|0,0,0\rangle\langle 0,0,0|-1)H^{\otimes \max(m,n)} \otimes H \otimes I_2)]$$

$$= I_2^{\otimes p} \otimes [\sum_{k,l} 2\frac{|k,+,0\rangle\langle l,+,0|}{max(M,N)} - I] \qquad (37)$$

Then the overlap between these two states $|\Phi_A\rangle$ and $R|\Phi_B\rangle$ is given by,

$$\langle \Phi_A | R | \Phi_B \rangle = \langle 0, i, 0, 0 | H_c^{\otimes p} V_A^\dagger R V_B H_c^{\otimes p} | 0, j, 1, 0 \rangle = \frac{2 \sum_c A'_{ic} B'_{cj}}{max(M,N)P} = \frac{\sum_c A_{ic} B_{cj}}{max(M,N)P \|A\| \|B\|} \tag{38}$$

By construction it is proved the existence of $U(A,B)$ that can be defined without any isometry,

$$U(A, B) = H_c^{\otimes p} V_A^\dagger R V_B H_c^{\otimes p} \tag{39}$$

Once the correlation exchange matrix is determined, at step 210c, a Fock matrix is computed by adding the core Hamiltonian matrix, the density matrix, and the correlation exchange matrix. At step 210d, qubitized diagonalization of the Fock matrix is performed to obtain an updated density matrix. The updated density matrix is used in a subsequent iteration. Step 210 is performed iteratively until the convergence criteria is satisfied to get a final density matrix. The convergence criteria is satisfied when norm of difference between the density matrix of a current iteration and the density matrix of a previous iteration is lesser than a pre-defined tolerance value. Finally, at step 212, the one or more classical hardware processors are configured to utilize the final density matrix of the chemical compound to extract the one or more properties of the chemical compound. The final density matrix thus obtained enables computation of one or more properties such as, (i) HOMO-LUMO gap from the energies of the highest occupied Kohn Sham orbital and lowest unoccupied orbital, (ii) the charge distribution from integrating the electronic density in regions around the different atoms, where the electronic density is in turn obtained from the density matrix, and (iii) dipole moment from the expectation value of the perturbation electric field operator with respect to the density matrix.

EXPERIMENTAL RESULTS

The J matrix computation was performed on classical processor using conventional methods and quantum processor. Also, the DFT was implemented through GGA using classical processors and quantum processors. Classical complexity is measured in terms of space and time complexity. Similarly, quantum complexity is measured in terms of number of qubits and gate complexity. The results of J-matrix computations and correlation exchange matrix computations are recorded in table 1.

Table 1

| Quantity | Classical Complexity | | Quantum Complexity | |
|---|---|---|---|---|
| | Space | Time | No. of Qubits | Gate complexity |
| J(AO) | $O(N_h^4)$ | $O(N_h^4)$ | 4 log $N_b$ + 2 | 8 log $N_b$ |
| J(DF) | $O(N_b^2 Naux)$ | $O(N_b^2 Naux)$ | 2 log $N_b$ + log $Naux$ | 4 log $N_b$ + 2 log $Naux$ |
| J(sN) | $O(N_b^2 N_g)$ | $O(N_b^2 N_g)$ | 2 log$N_b$ + log $N_g$ | 4 log $N_b$ + 2 log $N_g$ |
| $V_{xc}[\rho, \nabla\rho](GGA)$ | $O(N^2 N_g)$ | $O(N^2 N_g)$ | $log\ N + logN_g$ | $4(logN + logN_g)N_b N_g$ |

The classical and quantum resources needed to compute the J matrix using the AO integral approach, the Density fitting (DF) approach and the semi-numerical exchange (sN) approach are shown in Table 1. For all these three types of methods to compute J matrix the Quantum circuits of same type as given in the present disclosure is applicable. For the AO integral approach the number of qubits needed to encode the electronic integrals is 4log Nb+2, where Nb=Nao is the number of basis functions. On the other hand for the classical approach the space complexity is $O(N_b^4)$ to store $N_b^4$ numbers. Log is clearly a sublinear memory scaling on the other hand the polynomial scaling involved is steep in classical approach. The gate complexity needed for computing the J matrix stems from two sources, one is the diffusion quantum circuit block and the multiqubit gates needed to encode the integrals and the density matrix. The first component from the diffusion quantum circuit block leads to the gate complexity 8 log $N_b$. The second component depends on the molecular system, and the gate complexity for that component depends on the floating-point precision with which we are encoding the integrals associated with the molecule. In comparison the classical time complexity scales as $O(N_b^4)$.

For the Density fitting approach the number of qubits needed to encode the electronic integrals is 2 log $N_b$ + *log Naux* , where *Nb = Nao* is the number of basis functions and *Naux* number of auxilliarly basis vectors. On the other hand for the classical approach the space complexity is $O(N_b^2 Naux)$ to store that many numbers. Log is clearly a sublinear memory scaling on the other hand the polynomial scaling involved is steep in classical approach. The gate complexity needed for computing the J matrix stems from two sources, one is the diffusion quantum circuit block and the multiqubit gates needed to encode the integrals and the density matrix. The first component from the diffusion quantum circuit block leads to the gate complexity 4 log $N_b$ + 2 log *Naux*. The second component depends on the molecular system, and the gate complexity for that component depends on the floating-point precision with which we are encoding the integrals associated with the molecule. In comparison the base classical time complexity scales as $O(N_b^2 Naux)$.

For the semi numerical engine approach the number of qubits needed to encode the electronic integrals is 2 log $N_b$ + log $N_g$, where *Nb = Nao* is the number of basis functions and Ng number of grid points. On the other hand for the classical approach the space complexity is $O(N_b^2 N_g)$ to store that many numbers. Log is clearly a sublinear memory scaling on the other hand the polynomial scaling involved is steep in classical approach. The gate complexity needed for computing the J matrix stems from two sources, one is the diffusion quantum circuit block and the multiqubit gates needed to encode the integrals and the density matrix. The first component from the diffusion quantum circuit block leads to the gate complexity 4 log $N_b$ + 2 log $N_g$. The second component depends on the molecular system, and the gate complexity for that component depends on the floating-point precision with which we are encoding the integrals associated with the molecule. In comparison the base classical time complexity scales as $O(N_b^2 N_g)$.

For the GGA protocol the correlation exchange matrix is a function of the electronic density and its gradient. For computing the electronic density, the number of qubits needed to encode the electronic density is given by *log N + log Ng,* where *Ng* is the number of points on numerical grid and *N* is number of basis functions. Additionally, 2 ancilla qubits are needed to encode the different components of the electronic density and its gradient. The gate complexity is associated with the diffusion operator that enables the multiplication between the collocation matrix, the gradient of the collocation matrix and the density matrix. And it scales logarithmically with the number of basis functions. Classically to store the integrals is $O(N^2 N_g)$, and the time complexity is $O(N^2 N_g)$.

The written description describes the subject matter herein to enable any person skilled in the art to make and use the embodiments. The scope of the subject matter embodiments is defined by the claims.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with the scope of disclosed embodiments being indicated by the following claims.

**Claims**

1. A quantum simulation method (200), performed by a system comprising one or more classical hardware processors and a plurality of unentangled Quantum Processor Units, QPUs, wherein the one or more classical hardware processors are communicably coupled to the plurality of unentangled QPUs by communication interfaces, wherein the quantum simulation method comprises:

    receiving, via the one or more classical hardware processors, a chemical compound whose one or more properties are to be extracted, wherein the chemical compound is one of (i) a molecule, and (ii) a solid (202);
    obtaining, via the one or more classical hardware processors, atomic coordinates of each of a plurality of atoms present in the chemical compound (204);
    determining, via the one or more classical hardware processors, a plurality of electron integrals, a core Hamiltonian matrix, and a collocation matrix from the atomic coordinates of each of the plurality of atoms, wherein the collocation matrix is a rectangular matrix of dimension Ng and NaO, and wherein Ng represents a number of points on a numerical grid, and NaO represents a number of basis functions (206);
    determining, via the plurality of unentangled QPUs, a density matrix of the chemical compound from the core Hamiltonian matrix wherein the density matrix is constructed using (i) a single particles unitary rotation matrix, obtained by diagonalizing the core Hamiltonian matrix, and (ii) electron occupancies (208); and
    iteratively updating, via the plurality of unentangled QPUs, the density matrix by computing a Fock matrix until a convergence criteria is met, wherein iteratively updating the density matrix at each iteration comprises (210):

        computing a direct, J, matrix from the density matrix based on a subset of electron integrals (210a);
        determining a correlation exchange matrix from the collocation matrix for generalized gradient approximation by (210b),

            encoding a gradient of the collocation matrix by processing a set of collocation matrices, wherein the set of collocation matrices are encoded for a set of positions (x+/-delta,y,z),(x,y+/-delta,z), (x,y,z+/-delta) for the number of basis functions at all (x,y,z) positions using a first quantum circuit component, a second quantum circuit component, a third quantum circuit component on (a) control qubits composed of a first set of qubits and a second set of qubits, and (b) a plurality of ancilla qubits wherein the first set of qubits is associated with the number of points on the numerical grid, the second set of qubits is associated with the number of basis functions, and the plurality of ancilla qubits are used to store numerical entries for the set of collocation matrices (210b1);
            encoding an electronic density by performing a sequential matrix multiplication of the collocation matrix, the density matrix, and the collocation matrix (210b2);
            computing an electronic density gradient from the collocation matrix, the density matrix, and the gradient of the collocation matrix by composing a fourth quantum circuit component and a fifth quantum circuit component using at least one ancilla qubit from the plurality of ancilla qubits (210b3);
            computing a derivative of an electronic energy density with respect to the electronic density by performing a quantum signal processing sequence on a sixth quantum circuit, wherein the sixth quantum circuit component encodes the electronic density and the electronic density gradient (210b4);
            encoding a Z-matrix by composing the sixth quantum circuit component and the collocation matrix (210b5); and
            encoding a correlation exchange matrix by composing the Z-matrix with the collocation matrix (210b6);

        determining the Fock matrix by combining the core Hamiltonian matrix, the density matrix, and the correlation exchange matrix (210c); and
        updating the density matrix by diagonalizing the Fock matrix (210d); and

    utilizing, via the one or more classical hardware processors, the updated density matrix of the chemical compound, to extract the one or more properties of the chemical compound (212).

2. The method of claim 1, wherein encoding the gradient of the collocation matrix comprises:

encoding, via the plurality of unentangled QPUs, a first subset of collocation matrices for a first set of grid points ((+delta x, 0,0) and (-delta x,0,0)) to obtain an X-component of the gradient of the collocation matrix using a first quantum circuit component;
encoding, via the plurality of unentangled QPUs, a second subset of collocation matrices for a second set of grid points ((0, +delta y, 0) and (0, -delta y, 0)) to obtain a Y-component of the gradient of the collocation matrix using a second quantum circuit component;
encoding, via the plurality of unentangled QPUs, a third subset of collocation matrices for a set of grid points ((0,0, +delta z) and (0,0, -delta z)) to obtain a Z-component of the gradient of the collocation matrix using a third quantum circuit component; and
composing, via the plurality of unentangled QPUs, the first quantum circuit component, the second quantum circuit component, and the third quantum circuit component to obtain the gradient of the collocation matrix.

3. The method of claim 1, wherein,

the fourth quantum circuit component sequentially encodes the collocation matrix, the density matrix, and the gradient of the collocation matrix, wherein the density matrix is encoded on control qubits composed of the second set of qubits and at least one ancilla qubits from the plurality of ancilla qubits, and
the fifth quantum circuit component sequentially encodes the gradient of the collocation matrix, the density matrix, and the collocation matrix.

4. The method of claim 1, wherein, the (i) control qubits composed of the first set of qubits and the second set of qubits, and (ii) the plurality of ancilla qubits, is mathematically represented as:

$$\log Ng + \log Nao + 1 + 2 \text{ qubits}$$

wherein log Ng represents the first set of qubits, log Nao represents the second set of qubits, and 1+2 qubits represent the plurality of ancilla qubits.

5. The method of claim 1, wherein the convergence criteria is satisfied when a norm of a difference between the density matrix of a current iteration and the density matrix of a previous iteration is lesser than a pre-defined tolerance value.

6. A system (100) comprising:
one or more classical hardware processors (108) and a plurality of unentangled Quantum Processor Units, QPUs, (122), wherein the one or more classical hardware processors (108) are communicably coupled to the plurality of unentangled QPUs (122) by one or more communication interfaces (106), wherein the one or more classical hardware processors are operatively coupled to at least one memory (110) storing programmed instructions and one or more Input/Output (I/O) interfaces (116); and the plurality of unentangled quantum processors (122) are operatively coupled to the at least one quantum memory (124), wherein the one or more classical hardware processors (108) and the plurality of unentangled QPUs (122) are configured by the programmed instructions to:

receive, via the one or more classical hardware processors (108), a chemical compound whose one or more properties are to be extracted, wherein the chemical compound is one of (i) a molecule, and (ii) a solid;
obtain, via the one or more classical hardware processors (108), atomic coordinates of each of a plurality of atoms present in the chemical compound;
determine, via the one or more classical hardware processors (108), a plurality of electron integrals, a core Hamiltonian matrix, and a collocation matrix from the atomic coordinates of each of the plurality of atoms, wherein the collocation matrix is a rectangular matrix of dimension Ng and NaO, and wherein Ng represents a number of points on a numerical grid, and NaO represents a number of basis functions;
determine, via the plurality of unentangled QPUs (122), a density matrix of the chemical compound from the core Hamiltonian matrix wherein the density matrix is constructed using (i) a single particles unitary rotation matrix, obtained by diagonalizing the core Hamiltonian matrix, and (ii) electron occupancies; and
iteratively update, via the plurality of unentangled QPUs (122), the density matrix by computing a Fock matrix until a convergence criteria is met, wherein iteratively updating the density matrix at each iteration comprises:

compute a direct, J, matrix from the density matrix based on a subset of electron integrals;

determine a correlation exchange matrix from the collocation matrix for generalized gradient approximation by,

encoding a gradient of the collocation matrix by processing a set of collocation matrices, wherein the set of collocation matrices are encoded for a set of positions (x+/-delta,y,z),(x,y+/-delta,z), (x,y,z+/-delta) for the number of basis functions at all (x,y,z) positions using a first quantum circuit component, a second quantum circuit component, a third quantum circuit component on (a) control qubits composed of a first set of qubits and a second set of qubits, and (b) a plurality of ancilla qubits wherein the first set of qubits is associated with the number of points on the numerical grid, the second set of qubits is associated with the number of basis functions, and the plurality of ancilla qubits are used to store numerical entries for the set of collocation matrices;

encoding an electronic density by performing a sequential matrix multiplication of the collocation matrix, the density matrix, and the collocation matrix;

computing an electronic density gradient from the collocation matrix, the density matrix, and the gradient of the collocation matrix by composing a fourth quantum circuit component and a fifth quantum circuit component using at least one ancilla qubit from the plurality of ancilla qubits;

computing a derivative of an electronic energy density with respect to the electronic density by performing a quantum signal processing sequence on a sixth quantum circuit, wherein the sixth quantum circuit component encodes the electronic density and the electronic density gradient;

encoding a Z-matrix by composing the sixth quantum circuit component and the collocation matrix; and

encoding a correlation exchange matrix by composing the Z-matrix with the collocation matrix;

determining the Fock matrix by combining the core Hamiltonian matrix, the density matrix, and the correlation exchange matrix; and

updating the density matrix by diagonalizing the Fock matrix; and utilize, via the one or more classical hardware processors (108), the updated density matrix of the chemical compound, to extract the one or more properties of the chemical compound.

7. The system of claim 6, wherein encoding the gradient of the collocation matrix comprises:

encoding a first subset of collocation matrices for a first set of grid points ((+delta x, 0,0) and (-delta x,0,0)) to obtain an X-component of the gradient of the collocation matrix using a first quantum circuit component;

encoding a second subset of collocation matrices for a second set of grid points ((0, +delta y, 0) and (0, -delta y, 0)) to obtain a Y-component of the gradient of the collocation matrix using a second quantum circuit component;

encoding a third subset of collocation matrices for a set of grid points ((0,0, +delta z) and (0,0, -delta z)) to obtain a Z-component of the gradient of the collocation matrix using a third quantum circuit component; and

composing the first quantum circuit component, the second quantum circuit component, and the third quantum circuit component to obtain the gradient of the collocation matrix.

8. The system of claim 6, wherein,

the fourth quantum circuit component sequentially encodes the collocation matrix, the density matrix, and the gradient of the collocation matrix, wherein the density matrix is encoded on control qubits composed of the second set of qubits and at least one ancilla qubits from the plurality of ancilla qubits, and

the fifth quantum circuit component sequentially encodes the gradient of the collocation matrix, the density matrix, and the collocation matrix.

9. The system of claim 6, wherein, the (i) control qubits composed of the first set of qubits and the second set of qubits, and (ii) the plurality of ancilla qubits, is mathematically represented as:

$$\log Ng + \log Nao + 1+2 \text{ qubits}$$

wherein log Ng represents the first set of qubits, log Nao represents the second set of qubits, and 1+2 qubits represent the plurality of ancilla qubits.

10. The system of claim 6, wherein the convergence criteria is satisfied as a norm of a difference between the density matrix of a current iteration and the density matrix of a previous iteration is lesser than a pre-defined tolerance value.

11. One or more non-transitory machine-readable information storage mediums comprising one or more instructions which when executed by one or more classical hardware processors and a plurality of unentangled quantum processors, QPUs, wherein the one or more classical hardware processors are communicably coupled to the plurality of unentangled QPUs by communication interfaces, cause:

receiving, via the one or more classical hardware processors, a chemical compound whose one or more properties are to be extracted, wherein the chemical compound is one of (i) a molecule, and (ii) a solid;

obtaining, via the one or more classical hardware processors, atomic coordinates of each of a plurality of atoms present in the chemical compound;

determining, via the one or more classical hardware processors, a plurality of electron integrals, a core Hamiltonian matrix, and a collocation matrix from the atomic coordinates of each of the plurality of atoms, wherein the collocation matrix is a rectangular matrix of dimension $Ng$ and $NaO$, and wherein $Ng$ represents a number of points on a numerical grid, and $NaO$ represents a number of basis functions;

determining, via the plurality of unentangled QPUs, a density matrix of the chemical compound from the core Hamiltonian matrix wherein the density matrix is constructed using (i) a single particles unitary rotation matrix, obtained by diagonalizing the core Hamiltonian matrix, and (ii) electron occupancies; and

iteratively updating, via the plurality of unentangled QPUs, the density matrix by computing a Fock matrix until a convergence criteria is met, wherein iteratively updating the density matrix at each iteration comprises:

computing a direct, J, matrix from the density matrix based on a subset of electron integrals;

determining a correlation exchange matrix from the collocation matrix for generalized gradient approximation by,

encoding a gradient of the collocation matrix by processing a set of collocation matrices, wherein the set of collocation matrices are encoded for a set of positions $(x+/-delta,y,z),(x,y+/-delta,z), (x,y,z+/-delta)$ for the number of basis functions at all $(x,y,z)$ positions using a first quantum circuit component, a second quantum circuit component, a third quantum circuit component on (a) control qubits composed of a first set of qubits and a second set of qubits, and (b) a plurality of ancilla qubits wherein the first set of qubits is associated with the number of points on the numerical grid, the second set of qubits is associated with the number of basis functions, and the plurality of ancilla qubits are used to store numerical entries for the set of collocation matrices;

encoding an electronic density by performing a sequential matrix multiplication of the collocation matrix, the density matrix, and the collocation matrix;

computing an electronic density gradient from the collocation matrix, the density matrix, and the gradient of the collocation matrix by composing a fourth quantum circuit component and a fifth quantum circuit component using at least one ancilla qubit from the plurality of ancilla qubits;

computing a derivative of an electronic energy density with respect to the electronic density by performing a quantum signal processing sequence on a sixth quantum circuit, wherein the sixth quantum circuit component encodes the electronic density and the electronic density gradient;

encoding a Z-matrix by composing the sixth quantum circuit component and the collocation matrix; and

encoding a correlation exchange matrix by composing the Z-matrix with the collocation matrix;

determining the Fock matrix by combining the core Hamiltonian matrix, the density matrix, and the correlation exchange matrix; and

updating the density matrix by diagonalizing the Fock matrix; and

utilizing, via the one or more classical hardware processors, the updated density matrix of the chemical compound, to extract the one or more properties of the chemical compound.

12. The one or more non-transitory machine-readable information storage mediums of claim 11, wherein encoding the gradient of the collocation matrix comprises:

encoding, via the plurality of unentangled QPUs, a first subset of collocation matrices for a first set of grid points $((+delta\ x, 0,0)$ and $(-delta\ x,0,0))$ to obtain an X-component of the gradient of the collocation matrix using a first quantum circuit component;

encoding, via the plurality of unentangled QPUs, a second subset of collocation matrices for a second set of grid points $((0, +delta\ y, 0)$ and $(0, -delta\ y, 0))$ to obtain a Y-component of the gradient of the collocation matrix using a second quantum circuit component;

encoding, via the plurality of unentangled QPUs, a third subset of collocation matrices for a set of grid points ((0,0, +delta z) and (0,0, -delta z)) to obtain a Z-component of the gradient of the collocation matrix using a third quantum circuit component; and

composing, via the plurality of unentangled QPUs, the first quantum circuit component, the second quantum circuit component, and the third quantum circuit component to obtain the gradient of the collocation matrix.

13. The one or more non-transitory machine-readable information storage mediums of claim 11, wherein,

the fourth quantum circuit component sequentially encodes the collocation matrix, the density matrix, and the gradient of the collocation matrix, wherein the density matrix is encoded on control qubits composed of the second set of qubits and at least one ancilla qubits from the plurality of ancilla qubits, and

the fifth quantum circuit component sequentially encodes the gradient of the collocation matrix, the density matrix, and the collocation matrix.

14. The one or more non-transitory machine-readable information storage mediums of claim 11, wherein, the (i) control qubits composed of the first set of qubits and the second set of qubits, and (ii) the plurality of ancilla qubits, is mathematically represented as:

$$\log Ng + \log Nao + 1+2 \text{ qubits}$$

wherein log Ng represents the first set of qubits, log Nao represents the second set of qubits, and 1+2 qubits represent the plurality of ancilla qubits.

15. The one or more non-transitory machine-readable information storage mediums of claim 11, wherein the convergence criteria is satisfied when a norm of a difference between the density matrix of a current iteration and the density matrix of a previous iteration is lesser than a pre-defined tolerance value.

**Patentansprüche**

1. Quantensimulationsverfahren (200), das von einem System durchgeführt wird, das einen oder mehrere klassische Hardwareprozessoren und eine Mehrzahl von nicht verschränkten Quantenprozessoreinheiten, QPUs, umfasst, wobei der eine oder die mehreren klassischen Hardwareprozessoren durch Kommunikationsschnittstellen kommunikativ mit der Mehrzahl von nicht verschränkten QPUs gekoppelt sind, wobei das Quantensimulationsverfahren Folgendes umfasst:

Empfangen, über den einen oder die mehreren klassischen Hardwareprozessoren, einer chemischen Verbindung, deren eine oder mehrere Eigenschaften extrahiert werden sollen, wobei die chemische Verbindung eines von (i) einem Molekül und (ii) einem Feststoff (202) ist;

Erhalten, über den einen oder die mehreren klassischen Hardwareprozessoren, von Atomkoordinaten von jedem einer Mehrzahl von Atomen, die in der chemischen Verbindung vorhanden sind (204);

Bestimmen, über den einen oder die mehreren klassischen Hardwareprozessoren, einer Mehrzahl von Elektronenintegralen, einer Hamilton-Kernmatrix und einer Kollokationsmatrix aus den Atomkoordinaten von jedem der Mehrzahl von Atomen, wobei die Kollokationsmatrix eine rechteckige Matrix der Dimension Ng und NaO ist, und wobei Ng eine Anzahl von Punkten auf einem numerischen Gitter darstellt und NaO eine Anzahl von Basisfunktionen darstellt (206);

Bestimmen, über die Mehrzahl von nicht verschränkten QPUs, einer Dichtematrix der chemischen Verbindung aus der Hamilton-Kernmatrix, wobei die Dichtematrix unter Verwendung von (i) einer einheitlichen Einzelteilchen-Rotationsmatrix, die durch Diagonalisieren der Hamilton-Kernmatrix erhalten wird, und (ii) Elektronenbelegungen konstruiert wird (208); und

iteratives Aktualisieren, über die Mehrzahl von nicht verschränkten QPUs, der Dichtematrix durch Berechnen einer Fock-Matrix, bis ein Konvergenzkriterium erfüllt ist, wobei das iterative Aktualisieren der Dichtematrix bei jeder Iteration Folgendes umfasst (210):

Berechnen einer direkten, J, Matrix aus der Dichtematrix basierend auf einer Teilmenge von Elektronenintegralen (210a);

Bestimmen einer Korrelationsaustauschmatrix aus der Kollokationsmatrix für eine generalisierte Gradientenapproximation durch (210b),

Codieren eines Gradienten der Kollokationsmatrix durch Verarbeiten eines Satzes von Kollokationsmatrizen, wobei der Satz von Kollokationsmatrizen für einen Satz von Positionen (x+/-delta,y,z), (x,y+/-delta,z), (x,y,z+/-delta) für die Anzahl von Basisfunktionen an allen (x,y,z) Positionen unter Verwendung einer ersten Quantenschaltungskomponente, einer zweiten Quantenschaltungskomponente, einer dritten Quantenschaltungskomponente auf (a) Steuer-Qubits, die aus einem ersten Satz von Qubits und einem zweiten Satz von Qubits zusammengesetzt sind, und (b) einer Mehrzahl von Hilfsqubits codiert wird, wobei der erste Satz von Qubits mit der Anzahl von Punkten auf dem numerischen Gitter assoziiert ist, der zweite Satz von Qubits mit der Anzahl von Basisfunktionen assoziiert ist und die Mehrzahl von Hilfsqubits verwendet wird, um numerische Einträge für den Satz von Kollokationsmatrizen zu speichern (210b1);

Codieren einer elektronischen Dichte durch Durchführen einer sequentiellen Matrixmultiplikation der Kollokationsmatrix, der Dichtematrix und der Kollokationsmatrix (210b2);

Berechnen eines elektronischen Dichtegradienten aus der Kollokationsmatrix, der Dichtematrix und dem Gradienten der Kollokationsmatrix durch Zusammensetzen einer vierten Quantenschaltungskomponente und einer fünften Quantenschaltungskomponente unter Verwendung mindestens eines Hilfsqubits aus der Mehrzahl von Hilfsqubits (210b3);

Berechnen einer Ableitung einer elektronischen Energiedichte in Bezug auf die elektronische Dichte durch Durchführen einer Quantensignalverarbeitungssequenz an einer sechsten Quantenschaltung, wobei die sechste Quantenschaltungskomponente die elektronische Dichte und den elektronischen Dichtegradienten codiert (210b4);

Codieren einer Z-Matrix durch Zusammensetzen der sechsten Quantenschaltungskomponente und der Kollokationsmatrix (210b5); und

Codieren einer Korrelationsaustauschmatrix durch Zusammensetzen der Z-Matrix mit der Kollokationsmatrix (210b6);

Bestimmen der Fock-Matrix durch Kombinieren der Hamilton-Kernmatrix, der Dichtematrix und der Korrelationsaustauschmatrix (210c); und

Aktualisieren der Dichtematrix durch Diagonalisieren der Fock-Matrix (210d); und

Verwenden, über den einen oder die mehreren klassischen Hardwareprozessoren, der aktualisierten Dichtematrix der chemischen Verbindung, um die eine oder die mehreren Eigenschaften der chemischen Verbindung zu extrahieren (212).

2. Verfahren nach Anspruch 1, wobei das Codieren des Gradienten der Kollokationsmatrix Folgendes umfasst:

Codieren, über die Mehrzahl von unverschränkten QPUs, einer ersten Teilmenge von Kollokationsmatrizen für einen ersten Satz von Gitterpunkten ((+delta x, 0,0) und (-delta x, 0,0)), um eine X-Komponente des Gradienten der Kollokationsmatrix unter Verwendung einer ersten Quantenschaltungskomponente zu erhalten;

Codieren, über die Mehrzahl von unverschränkten QPUs, einer zweiten Teilmenge von Kollokationsmatrizen für einen zweiten Satz von Gitterpunkten ((0, +delta y, 0) und (0, -delta y, 0)), um eine Y-Komponente des Gradienten der Kollokationsmatrix unter Verwendung einer zweiten Quantenschaltungskomponente zu erhalten;

Codieren, über die Mehrzahl von unverschränkten QPUs, einer dritten Teilmenge von Kollokationsmatrizen für einen Satz von Gitterpunkten ((0, 0, +delta z) und (0, 0, -delta z)), um eine Z-Komponente des Gradienten der Kollokationsmatrix unter Verwendung einer dritten Quantenschaltungskomponente zu erhalten; und

Zusammensetzen, über die Mehrzahl von unverschränkten QPUs, der ersten Quantenschaltungskomponente, der zweiten Quantenschaltungskomponente und der dritten Quantenschaltungskomponente, um den Gradienten der Kollokationsmatrix zu erhalten.

3. Verfahren nach Anspruch 1, wobei

die vierte Quantenschaltungskomponente die Kollokationsmatrix, die Dichtematrix und den Gradienten der Kollokationsmatrix sequenziell codiert, wobei die Dichtematrix auf Steuer-Qubits codiert wird, die aus dem zweiten Satz von Qubits und mindestens einem Hilfsqubit aus der Mehrzahl von Hilfsqubits zusammengesetzt sind, und

die fünfte Quantenschaltungskomponente den Gradienten der Kollokationsmatrix, die Dichtematrix und die Kollokationsmatrix sequenziell codiert.

4. Verfahren nach Anspruch 1, wobei die (i) Steuer-Qubits, die aus dem ersten Satz von Qubits und dem zweiten Satz von Qubits zusammengesetzt sind, und (ii) die Mehrzahl von Hilfsqubits mathematisch dargestellt werden als:

$$\log Ng + \log Nao + 1+2 \text{ Qubits,}$$

wobei log Ng den ersten Satz von Qubits darstellt, log Nao den zweiten Satz von Qubits darstellt und 1+2 Qubits die Mehrzahl von Hilfsqubits darstellen.

5. Verfahren nach Anspruch 1, wobei das Konvergenzkriterium erfüllt ist, wenn eine Norm einer Differenz zwischen der Dichtematrix einer aktuellen Iteration und der Dichtematrix einer vorherigen Iteration kleiner als ein vordefinierter Toleranzwert ist.

6. System (100), umfassend:

einen oder mehrere klassische Hardwareprozessoren (108) und eine Mehrzahl von nicht verschränkten Quantenprozessoreinheiten, QPUs, (122), wobei der eine oder die mehreren klassischen Hardwareprozessoren (108) durch eine oder mehrere Kommunikationsschnittstellen (106) kommunikativ mit der Mehrzahl von nicht verschränkten QPUs (122) gekoppelt sind, wobei der eine oder die mehreren klassischen Hardwareprozessoren betriebsfähig mit mindestens einem Speicher (110) gekoppelt sind, der programmierte Anweisungen und eine oder mehrere Eingabe/Ausgabe(E/A)-Schnittstellen (116) speichert; und die Mehrzahl von nicht verschränkten Quantenprozessoren (122) betriebsfähig mit dem mindestens einen Quantenspeicher (124) gekoppelt sind, wobei der eine oder die mehreren klassischen Hardwareprozessoren (108) und die Mehrzahl von nicht verschränkten QPUs (122) durch die programmierten Anweisungen konfiguriert sind zum:

Empfangen, über den einen oder die mehreren klassischen Hardwareprozessoren (108), einer chemischen Verbindung, deren eine oder mehrere Eigenschaften extrahiert werden sollen, wobei die chemische Verbindung eines von (i) einem Molekül und (ii) einem Feststoff ist;
Erhalten, über den einen oder die mehreren klassischen Hardwareprozessoren (108), von Atomkoordinaten von jedem einer Mehrzahl von Atomen, die in der chemischen Verbindung vorhanden sind;
Bestimmen, über den einen oder die mehreren klassischen Hardwareprozessoren (108), einer Mehrzahl von Elektronenintegralen, einer Hamilton-Kernmatrix und einer Kollokationsmatrix aus den Atomkoordinaten von jedem der Mehrzahl von Atomen, wobei die Kollokationsmatrix eine rechteckige Matrix der Dimension Ng und NaO ist, und wobei Ng eine Anzahl von Punkten auf einem numerischen Gitter darstellt und NaO eine Anzahl von Basisfunktionen darstellt;
Bestimmen, über die Mehrzahl von nicht verschränkten QPUs (122), einer Dichtematrix der chemischen Verbindung aus der Hamilton-Kernmatrix, wobei die Dichtematrix unter Verwendung von (i) einer einheitlichen Einzelteilchen-Rotationsmatrix, die durch Diagonalisieren der Hamilton-Kernmatrix erhalten wird, und (ii) Elektronenbelegungen konstruiert wird; und
iteratives Aktualisieren, über die Mehrzahl von nicht verschränkten QPUs (122), der Dichtematrix durch Berechnen einer Fock-Matrix, bis ein Konvergenzkriterium erfüllt ist, wobei das iterative Aktualisieren der Dichtematrix bei jeder Iteration Folgendes umfasst:

Berechnen einer direkten, J, Matrix aus der Dichtematrix basierend auf einer Teilmenge von Elektronenintegralen;
Bestimmen einer Korrelationsaustauschmatrix aus der Kollokationsmatrix für eine generalisierte Gradientenapproximation durch,
Codieren eines Gradienten der Kollokationsmatrix durch Verarbeiten eines Satzes von Kollokationsmatrizen, wobei der Satz von Kollokationsmatrizen für einen Satz von Positionen (x+/-delta,y,z), (x,y+/-delta,z), (x,y,z+/-delta) für die Anzahl von Basisfunktionen an allen (x,y,z) Positionen unter Verwendung einer ersten Quantenschaltungskomponente, einer zweiten Quantenschaltungskomponente, einer dritten Quantenschaltungskomponente auf (a) Steuer-Qubits, die aus einem ersten Satz von Qubits und einem zweiten Satz von Qubits zusammengesetzt sind, und (b) einer Mehrzahl von Hilfsqubits codiert wird, wobei der erste Satz von Qubits mit der Anzahl von Punkten auf dem numerischen Gitter assoziiert ist, der zweite Satz von Qubits mit der Anzahl von Basisfunktionen assoziiert ist und die Mehrzahl von Hilfsqubits verwendet wird, um numerische Einträge für den Satz von Kollokationsmatrizen zu speichern;
Codieren einer elektronischen Dichte durch Durchführen einer sequentiellen Matrixmultiplikation der Kollokationsmatrix, der Dichtematrix und der Kollokationsmatrix;

Berechnen eines elektronischen Dichtegradienten aus der Kollokationsmatrix, der Dichtematrix und dem Gradienten der Kollokationsmatrix durch Zusammensetzen einer vierten Quantenschaltungskomponente und einer fünften Quantenschaltungskomponente unter Verwendung mindestens eines Hilfsqubits aus der Mehrzahl von Hilfsqubits;

Berechnen einer Ableitung einer elektronischen Energiedichte in Bezug auf die elektronische Dichte durch Durchführen einer Quantensignalverarbeitungssequenz an einer sechsten Quantenschaltung, wobei die sechste Quantenschaltungskomponente die elektronische Dichte und den elektronischen Dichtegradienten codiert;

Codieren einer Z-Matrix durch Zusammensetzen der sechsten Quantenschaltungskomponente und der Kollokationsmatrix; und

Codieren einer Korrelationsaustauschmatrix durch Zusammensetzen der Z-Matrix mit der Kollokationsmatrix;

Bestimmen der Fock-Matrix durch Kombinieren der Hamilton-Kernmatrix, der Dichtematrix und der Korrelationsaustauschmatrix; und

Aktualisieren der Dichtematrix durch Diagonalisieren der Fock-Matrix; und

Verwenden, über den einen oder die mehreren klassischen Hardwareprozessoren (108), der aktualisierten Dichtematrix der chemischen Verbindung, um die eine oder die mehreren Eigenschaften der chemischen Verbindung zu extrahieren.

7. System nach Anspruch 6, wobei das Codieren des Gradienten der Kollokationsmatrix Folgendes umfasst:

Codieren einer ersten Teilmenge von Kollokationsmatrizen für einen ersten Satz von Gitterpunkten ((+delta x, 0,0) und (-delta x, 0,0)), um eine X-Komponente des Gradienten der Kollokationsmatrix unter Verwendung einer ersten Quantenschaltungskomponente zu erhalten;

Codieren einer zweiten Teilmenge von Kollokationsmatrizen für einen zweiten Satz von Gitterpunkten ((0, +delta y, 0) und (0, -delta y, 0)), um eine Y-Komponente des Gradienten der Kollokationsmatrix unter Verwendung einer zweiten Quantenschaltungskomponente zu erhalten;

Codieren einer dritten Teilmenge von Kollokationsmatrizen für einen Satz von Gitterpunkten ((0, 0, +delta z) und (0, 0, -delta z)), um eine Z-Komponente des Gradienten der Kollokationsmatrix unter Verwendung einer dritten Quantenschaltungskomponente zu erhalten; und

Zusammensetzen der ersten Quantenschaltungskomponente, der zweiten Quantenschaltungskomponente und der dritten Quantenschaltungskomponente, um den Gradienten der Kollokationsmatrix zu erhalten.

8. System nach Anspruch 6, wobei

die vierte Quantenschaltungskomponente die Kollokationsmatrix, die Dichtematrix und den Gradienten der Kollokationsmatrix sequenziell codiert, wobei die Dichtematrix auf Steuer-Qubits codiert wird, die aus dem zweiten Satz von Qubits und mindestens einem Hilfsqubit aus der Mehrzahl von Hilfsqubits zusammengesetzt sind, und

die fünfte Quantenschaltungskomponente den Gradienten der Kollokationsmatrix, die Dichtematrix und die Kollokationsmatrix sequenziell codiert.

9. System nach Anspruch 6, wobei die (i) Steuer-Qubits, die aus dem ersten Satz von Qubits und dem zweiten Satz von Qubits zusammengesetzt sind, und (ii) die Mehrzahl von Hilfsqubits mathematisch dargestellt werden als:

$$\log Ng + \log Nao + 1+2 \text{ Qubits,}$$

wobei log Ng den ersten Satz von Qubits darstellt, log Nao den zweiten Satz von Qubits darstellt und 1+2 Qubits die Mehrzahl von Hilfsqubits darstellen.

10. System nach Anspruch 6, wobei das Konvergenzkriterium erfüllt ist, wenn eine Norm einer Differenz zwischen der Dichtematrix einer aktuellen Iteration und der Dichtematrix einer vorherigen Iteration kleiner als ein vordefinierter Toleranzwert ist.

**11.** Ein oder mehrere nichtflüchtige maschinenlesbare Informationsspeichermedien, die eine oder mehrere Anweisungen umfassen, die, wenn sie von einem oder mehreren klassischen Hardwareprozessoren und einer Mehrzahl von nicht verschränkten Quantenprozessoren, QPUs, ausgeführt werden, wobei der eine oder die mehreren klassischen Hardwareprozessoren durch Kommunikationsschnittstellen kommunikativ mit der Mehrzahl von nicht verschränkten QPUs gekoppelt sind, Folgendes bewirken:

Empfangen, über den einen oder die mehreren klassischen Hardwareprozessoren, einer chemischen Verbindung, deren eine oder mehrere Eigenschaften extrahiert werden sollen, wobei die chemische Verbindung eines von (i) einem Molekül und (ii) einem Feststoff ist;

Erhalten, über den einen oder die mehreren klassischen Hardwareprozessoren, von Atomkoordinaten von jedem einer Mehrzahl von Atomen, die in der chemischen Verbindung vorhanden sind;

Bestimmen, über den einen oder die mehreren klassischen Hardwareprozessoren, einer Mehrzahl von Elektronenintegralen, einer Hamilton-Kernmatrix und einer Kollokationsmatrix aus den Atomkoordinaten von jedem der Mehrzahl von Atomen, wobei die Kollokationsmatrix eine rechteckige Matrix der Dimension Ng und NaO ist, und wobei Ng eine Anzahl von Punkten auf einem numerischen Gitter darstellt und NaO eine Anzahl von Basisfunktionen darstellt;

Bestimmen, über die Mehrzahl von nicht verschränkten QPUs, einer Dichtematrix der chemischen Verbindung aus der Hamilton-Kernmatrix, wobei die Dichtematrix unter Verwendung von (i) einer einheitlichen Einzelteilchen-Rotationsmatrix, die durch Diagonalisieren der Hamilton-Kernmatrix erhalten wird, und (ii) Elektronenbelegungen konstruiert wird; und

iteratives Aktualisieren, über die Mehrzahl von nicht verschränkten QPUs, der Dichtematrix durch Berechnen einer Fock-Matrix, bis ein Konvergenzkriterium erfüllt ist, wobei das iterative Aktualisieren der Dichtematrix bei jeder Iteration Folgendes umfasst:

Berechnen einer direkten, J, Matrix aus der Dichtematrix basierend auf einer Teilmenge von Elektronenintegralen;

Bestimmen einer Korrelationsaustauschmatrix aus der Kollokationsmatrix für eine generalisierte Gradientenapproximation durch,

Codieren eines Gradienten der Kollokationsmatrix durch Verarbeiten eines Satzes von Kollokationsmatrizen, wobei der Satz von Kollokationsmatrizen für einen Satz von Positionen (x+/-delta,y,z), (x,y+/-delta,z), (x,y,z+/-delta) für die Anzahl von Basisfunktionen an allen (x,y,z) Positionen unter Verwendung einer ersten Quantenschaltungskomponente, einer zweiten Quantenschaltungskomponente, einer dritten Quantenschaltungskomponente auf (a) Steuer-Qubits, die aus einem ersten Satz von Qubits und einem zweiten Satz von Qubits zusammengesetzt sind, und (b) einer Mehrzahl von Hilfsqubits codiert wird, wobei der erste Satz von Qubits mit der Anzahl von Punkten auf dem numerischen Gitter assoziiert ist, der zweite Satz von Qubits mit der Anzahl von Basisfunktionen assoziiert ist und die Mehrzahl von Hilfsqubits verwendet wird, um numerische Einträge für den Satz von Kollokationsmatrizen zu speichern;

Codieren einer elektronischen Dichte durch Durchführen einer sequentiellen Matrixmultiplikation der Kollokationsmatrix, der Dichtematrix und der Kollokationsmatrix;

Berechnen eines elektronischen Dichtegradienten aus der Kollokationsmatrix, der Dichtematrix und dem Gradienten der Kollokationsmatrix durch Zusammensetzen einer vierten Quantenschaltungskomponente und einer fünften Quantenschaltungskomponente unter Verwendung mindestens eines Hilfsqubits aus der Mehrzahl von Hilfsqubits;

Berechnen einer Ableitung einer elektronischen Energiedichte in Bezug auf die elektronische Dichte durch Durchführen einer Quantensignalverarbeitungssequenz an einer sechsten Quantenschaltung, wobei die sechste Quantenschaltungskomponente die elektronische Dichte und den elektronischen Dichtegradienten codiert;

Codieren einer Z-Matrix durch Zusammensetzen der sechsten Quantenschaltungskomponente und der Kollokationsmatrix; und

Codieren einer Korrelationsaustauschmatrix durch Zusammensetzen der Z-Matrix mit der Kollokationsmatrix;

Bestimmen der Fock-Matrix durch Kombinieren der Hamilton-Kernmatrix, der Dichtematrix und der Korrelationsaustauschmatrix; und

Aktualisieren der Dichtematrix durch Diagonalisieren der Fock-Matrix; und

Verwenden, über den einen oder die mehreren klassischen Hardwareprozessoren, der aktualisierten Dichtematrix der chemischen Verbindung, um die eine oder die mehreren Eigenschaften der chemischen Verbindung zu extrahieren.

12. Ein oder mehrere nichtflüchtige maschinenlesbare Informationsspeichermedien nach Anspruch 11, wobei das Codieren des Gradienten der Kollokationsmatrix Folgendes umfasst:

Codieren, über die Mehrzahl von unverschränkten QPUs, einer ersten Teilmenge von Kollokationsmatrizen für einen ersten Satz von Gitterpunkten ((+delta x, 0,0) und (-delta x, 0,0)), um eine X-Komponente des Gradienten der Kollokationsmatrix unter Verwendung einer ersten Quantenschaltungskomponente zu erhalten;
Codieren, über die Mehrzahl von unverschränkten QPUs, einer zweiten Teilmenge von Kollokationsmatrizen für einen zweiten Satz von Gitterpunkten ((0, +delta y, 0) und (0, -delta y, 0)), um eine Y-Komponente des Gradienten der Kollokationsmatrix unter Verwendung einer zweiten Quantenschaltungskomponente zu erhalten;
Codieren, über die Mehrzahl von unverschränkten QPUs, einer dritten Teilmenge von Kollokationsmatrizen für einen Satz von Gitterpunkten ((0, 0, +delta z) und (0, 0, -delta z)), um eine Z-Komponente des Gradienten der Kollokationsmatrix unter Verwendung einer dritten Quantenschaltungskomponente zu erhalten; und
Zusammensetzen, über die Mehrzahl von unverschränkten QPUs, der ersten Quantenschaltungskomponente, der zweiten Quantenschaltungskomponente und der dritten Quantenschaltungskomponente, um den Gradienten der Kollokationsmatrix zu erhalten.

13. Ein oder mehrere nichtflüchtige maschinenlesbare Informationsspeichermedien nach Anspruch 11, wobei

die vierte Quantenschaltungskomponente die Kollokationsmatrix, die Dichtematrix und den Gradienten der Kollokationsmatrix sequenziell codiert, wobei die Dichtematrix auf Steuer-Qubits codiert wird, die aus dem zweiten Satz von Qubits und mindestens einem Hilfsqubit aus der Mehrzahl von Hilfsqubits zusammengesetzt sind, und
die fünfte Quantenschaltungskomponente den Gradienten der Kollokationsmatrix, die Dichtematrix und die Kollokationsmatrix sequenziell codiert.

14. Ein oder mehrere nichtflüchtige maschinenlesbare Informationsspeichermedien nach Anspruch 11, wobei die (i) Steuer-Qubits, die aus dem ersten Satz von Qubits und dem zweiten Satz von Qubits zusammengesetzt sind, und (ii) die Mehrzahl von Hilfsqubits mathematisch dargestellt werden als:

$$\log Ng + \log Nao + 1+2 \text{ Qubits,}$$

wobei log Ng den ersten Satz von Qubits darstellt, log Nao den zweiten Satz von Qubits darstellt und 1+2 Qubits die Mehrzahl von Hilfsqubits darstellen.

15. Ein oder mehrere nichtflüchtige maschinenlesbare Informationsspeichermedien nach Anspruch 11, wobei das Konvergenzkriterium erfüllt ist, wenn eine Norm einer Differenz zwischen der Dichtematrix einer aktuellen Iteration und der Dichtematrix einer vorherigen Iteration kleiner als ein vordefinierter Toleranzwert ist.

**Revendications**

1. Procédé de simulation quantique (200), réalisé par un système comprenant un ou plusieurs processeurs matériels classiques et une pluralité d'unités de processeur quantique, QPU, non enchevêtrées, dans lequel les un ou plusieurs processeurs matériels classiques sont couplés de manière communicante à la pluralité de QPU non enchevêtrées par des interfaces de communication, dans lequel le procédé de simulation quantique comprend :

la réception, via les un ou plusieurs processeurs matériels classiques, d'un composé chimique dont une ou plusieurs propriétés doivent être extraites, dans lequel le composé chimique est l'un parmi (i) une molécule, et (ii) un solide (202) ;
l'obtention, via les un ou plusieurs processeurs matériels classiques, de coordonnées atomiques de chacun d'une pluralité d'atomes présents dans le composé chimique (204) ;
la détermination, via les un ou plusieurs processeurs matériels classiques, d'une pluralité d'intégrales d'électrons, d'une matrice hamiltonienne de coeur, et d'une matrice de colocation à partir des coordonnées atomiques de chacun de la pluralité d'atomes, dans lequel la matrice de colocation est une matrice rectangulaire de dimension Ng et NaO, et dans lequel Ng représente un nombre de points sur une grille numérique, et NaO représente un nombre de fonctions de base (206) ;

la détermination, via la pluralité de QPU non enchevêtrées, d'une matrice de densité du composé chimique à partir de la matrice hamiltonienne de coeur dans lequel la matrice de densité est construite en utilisant (i) une matrice de rotation unitaire de particules uniques, obtenue en diagonalisant la matrice hamiltonienne de coeur, et (ii) des occupations d'électrons (208) ; et

la mise à jour itérative, via la pluralité de QPU non enchevêtrées, de la matrice de densité en calculant une matrice de Fock jusqu'à ce qu'un critère de convergence soit satisfait, dans lequel la mise à jour itérative de la matrice de densité à chaque itération comprend (210) :

le calcul d'une matrice directe, J, à partir de la matrice de densité sur la base d'un sous-ensemble d'intégrales d'électrons (210a) ;

la détermination d'une matrice d'échange de corrélation à partir de la matrice de colocation pour une approximation de gradient généralisée par (210b),

le codage d'un gradient de la matrice de colocation en traitant un ensemble de matrices de colocation, dans lequel l'ensemble de matrices de colocation sont codées pour un ensemble de positions (x+/-delta,y,z), (x,y+/-delta,z), (x,y,z+/-delta) pour le nombre de fonctions de base à toutes les positions (x,y,z) en utilisant un premier composant de circuit quantique, un deuxième composant de circuit quantique, un troisième composant de circuit quantique sur (a) des qubits de commande composés d'un premier ensemble de qubits et d'un deuxième ensemble de qubits, et (b) une pluralité de qubits auxiliaires dans lequel le premier ensemble de qubits est associé au nombre de points sur la grille numérique, le deuxième ensemble de qubits est associé au nombre de fonctions de base, et la pluralité de qubits auxiliaires sont utilisés pour stocker des entrées numériques pour l'ensemble de matrices de colocation (210b1) ;

le codage d'une densité électronique en effectuant une multiplication matricielle séquentielle de la matrice de colocation, de la matrice de densité, et de la matrice de colocation (210b2) ;

le calcul d'un gradient de densité électronique à partir de la matrice de colocation, de la matrice de densité, et du gradient de la matrice de colocation en composant un quatrième composant de circuit quantique et un cinquième composant de circuit quantique en utilisant au moins un qubit auxiliaire parmi la pluralité de qubits auxiliaires (210b3) ;

le calcul d'une dérivée d'une densité d'énergie électronique par rapport à la densité électronique en effectuant une séquence de traitement de signal quantique sur un sixième circuit quantique, dans lequel le sixième composant de circuit quantique code la densité électronique et le gradient de densité électronique (210b4) ;

le codage d'une matrice Z en composant le sixième composant de circuit quantique et la matrice de colocation (210b5) ; et

le codage d'une matrice d'échange de corrélation en composant la matrice Z avec la matrice de colocation (210b6) ;

la détermination de la matrice de Fock en combinant la matrice hamiltonienne de coeur, la matrice de densité, et la matrice d'échange de corrélation (210c) ; et

la mise à jour de la matrice de densité en diagonalisant la matrice de Fock (210d) ; et

l'utilisation, via les un ou plusieurs processeurs matériels classiques, de la matrice de densité mise à jour du composé chimique, pour extraire les une ou plusieurs propriétés du composé chimique (212).

2. Procédé selon la revendication 1, dans lequel le codage du gradient de la matrice de colocation comprend :

le codage, via la pluralité de QPU non entremêlées, d'un premier sous-ensemble de matrices de colocation pour un premier ensemble de points de grille ((+delta x, 0,0) et (-delta x, 0,0)) pour obtenir un composant X du gradient de la matrice de colocation en utilisant un premier composant de circuit quantique ;

le codage, via la pluralité de QPU non entremêlées, d'un deuxième sous-ensemble de matrices de colocation pour un deuxième ensemble de points de grille ((0, +delta y, 0) et (0, -delta y, 0)) pour obtenir un composant Y du gradient de la matrice de colocation en utilisant un deuxième composant de circuit quantique ;

le codage, via la pluralité de QPU non entremêlées, d'un troisième sous-ensemble de matrices de colocation pour un ensemble de points de grille ((0, 0, +delta z) et (0, 0, - delta z)) pour obtenir un composant Z du gradient de la matrice de colocation en utilisant un troisième composant de circuit quantique ; et
la composition, via la pluralité de QPU non entremêlées, du premier composant de circuit quantique, du deuxième composant de circuit quantique, et du troisième composant de circuit quantique pour obtenir le gradient de la matrice de colocation.

**3.** Procédé selon la revendication 1, dans lequel,

le quatrième composant de circuit quantique code séquentiellement la matrice de colocation, la matrice de densité, et le gradient de la matrice de colocation, dans lequel la matrice de densité est codée sur des qubits de commande composés du deuxième ensemble de qubits et d'au moins un qubit auxiliaire parmi la pluralité de qubits auxiliaires, et
le cinquième composant de circuit quantique code séquentiellement le gradient de la matrice de colocation, la matrice de densité, et la matrice de colocation.

**4.** Procédé selon la revendication 1, dans lequel, les (i) qubits de commande composés du premier ensemble de qubits et du deuxième ensemble de qubits, et (ii) de la pluralité de qubits auxiliaires, sont représentés mathématiquement comme :

$$\log Ng + \log Nao + 1+2 \text{ qubits}$$

dans lequel log Ng représente le premier ensemble de qubits, log Nao représente le deuxième ensemble de qubits, et 1+2 qubits représentent la pluralité de qubits auxiliaires.

**5.** Procédé selon la revendication 1, dans lequel le critère de convergence est satisfait lorsqu'une norme d'une différence entre la matrice de densité d'une itération courante et la matrice de densité d'une itération précédente est inférieure à une valeur de tolérance prédéfinie.

**6.** Système (100) comprenant :

un ou plusieurs processeurs matériels classiques (108) et une pluralité d'unités de processeur quantique, QPU, non enchevêtrées (122), dans lequel les un ou plusieurs processeurs matériels classiques (108) sont couplés de manière communicante à la pluralité de QPU non enchevêtrées (122) par une ou plusieurs interfaces de communication (106), dans lequel les un ou plusieurs processeurs matériels classiques sont couplés de manière opérationnelle à au moins une mémoire (110) stockant des instructions programmées et une ou plusieurs interfaces d'entrée/sortie (E/S) (116) ; et la pluralité de processeurs quantiques non enchevêtrés (122) sont couplés de manière opérationnelle à l'au moins une mémoire quantique (124), dans lequel les un ou plusieurs processeurs matériels classiques (108) et la pluralité de QPU non enchevêtrées (122) sont configurés par les instructions programmées pour :

recevoir, via les un ou plusieurs processeurs matériels classiques (108), un composé chimique dont une ou plusieurs propriétés doivent être extraites, dans lequel le composé chimique est l'un parmi (i) une molécule, et (ii) un solide ;
obtenir, via les un ou plusieurs processeurs matériels classiques (108), des coordonnées atomiques de chacun d'une pluralité d'atomes présents dans le composé chimique ;
déterminer, via les un ou plusieurs processeurs matériels classiques (108), une pluralité d'intégrales d'électrons, une matrice hamiltonienne de coeur, et une matrice de colocation à partir des coordonnées atomiques de chacun de la pluralité d'atomes, dans lequel la matrice de colocation est une matrice rectangulaire de dimension Ng et NaO, et dans lequel Ng représente un nombre de points sur une grille numérique, et NaO représente un nombre de fonctions de base ;
déterminer, via la pluralité de QPU non enchevêtrées (122), une matrice de densité du composé chimique à partir de la matrice hamiltonienne de coeur dans lequel la matrice de densité est construite en utilisant (i) une matrice de rotation unitaire de particules uniques, obtenue en diagonalisant la matrice hamiltonienne de coeur, et (ii) des occupations d'électrons ; et
mettre à jour itérativement, via la pluralité de QPU non enchevêtrées (122), la matrice de densité en calculant une matrice de Fock jusqu'à ce qu'un critère de convergence soit satisfait, dans lequel la mise à jour itérative de la matrice de densité à chaque itération comprend :

le calcul d'une matrice directe, J, à partir de la matrice de densité sur la base d'un sous-ensemble d'intégrales d'électrons ;

la détermination d'une matrice d'échange de corrélation à partir de la matrice de colocation pour une approximation de gradient généralisée par,

le codage d'un gradient de la matrice de colocation en traitant un ensemble de matrices de colocation, dans lequel l'ensemble de matrices de colocation sont codées pour un ensemble de positions (x+/-delta,y,z), (x,y+/-delta,z), (x,y,z+/-delta) pour le nombre de fonctions de base à toutes les positions (x,y,z) en utilisant un premier composant de circuit quantique, un deuxième composant de circuit quantique, un troisième composant de circuit quantique sur (a) des qubits de commande composés d'un premier ensemble de qubits et d'un deuxième ensemble de qubits, et (b) une pluralité de qubits auxiliaires dans lequel le premier ensemble de qubits est associé au nombre de points sur la grille numérique, le deuxième ensemble de qubits est associé au nombre de fonctions de base, et la pluralité de qubits auxiliaires sont utilisés pour stocker des entrées numériques pour l'ensemble de matrices de colocation ;

le codage d'une densité électronique en effectuant une multiplication matricielle séquentielle de la matrice de colocation, la matrice de densité, et la matrice de colocation ;

le calcul d'un gradient de densité électronique à partir de la matrice de colocation, de la matrice de densité, et du gradient de la matrice de colocation en composant un quatrième composant de circuit quantique et un cinquième composant de circuit quantique en utilisant au moins un qubit auxiliaire parmi la pluralité de qubits auxiliaires ;

le calcul d'une dérivée d'une densité d'énergie électronique par rapport à la densité électronique en effectuant une séquence de traitement de signal quantique sur un sixième circuit quantique, dans lequel le sixième composant de circuit quantique code la densité électronique et le gradient de densité électronique ;

le codage d'une matrice Z en composant le sixième composant de circuit quantique et la matrice de colocation ; et

le codage d'une matrice d'échange de corrélation en composant la matrice Z avec la matrice de colocation ;

la détermination de la matrice de Fock en combinant la matrice hamiltonienne de coeur, la matrice de densité, et la matrice d'échange de corrélation ; et

la mise à jour de la matrice de densité en diagonalisant la matrice de Fock ; et

l'utilisation, via les un ou plusieurs processeurs matériels classiques (108), de la matrice de densité mise à jour du composé chimique, pour extraire les une ou plusieurs propriétés du composé chimique.

7. Système selon la revendication 6, dans lequel le codage du gradient de la matrice de colocation comprend :

le codage d'un premier sous-ensemble de matrices de colocation pour un premier ensemble de points de grille ((+delta x, 0,0) et (-delta x, 0,0)) pour obtenir un composant X du gradient de la matrice de colocation en utilisant un premier composant de circuit quantique ;

le codage d'un deuxième sous-ensemble de matrices de colocation pour un deuxième ensemble de points de grille ((0, +delta y, 0) et (0, -delta y, 0)) pour obtenir un composant Y du gradient de la matrice de colocation en utilisant un deuxième composant de circuit quantique ;

le codage d'un troisième sous-ensemble de matrices de colocation pour un ensemble de points de grille ((0, 0, +delta z) et (0, 0, -delta z)) pour obtenir un composant Z du gradient de la matrice de colocation en utilisant un troisième composant de circuit quantique ; et

la composition du premier composant de circuit quantique, du deuxième composant de circuit quantique, et du troisième composant de circuit quantique pour obtenir le gradient de la matrice de colocation.

8. Système selon la revendication 6, dans lequel,

le quatrième composant de circuit quantique code séquentiellement la matrice de colocation, la matrice de densité, et le gradient de la matrice de colocation, dans lequel la matrice de densité est codée sur des qubits de commande composés du deuxième ensemble de qubits et d'au moins un qubit auxiliaire parmi la pluralité de qubits auxiliaires, et

le cinquième composant de circuit quantique code séquentiellement le gradient de la matrice de colocation, la matrice de densité, et la matrice de colocation.

9. Système selon la revendication 6, dans lequel, les (i) qubits de commande composés du premier ensemble de qubits et du deuxième ensemble de qubits, et (ii) de la pluralité de qubits auxiliaires, sont représentés mathématiquement comme :

$$\log Ng + \log Nao + 1+2 \text{ qubits}$$

dans lequel log Ng représente le premier ensemble de qubits, log Nao représente le deuxième ensemble de qubits, et 1+2 qubits représentent la pluralité de qubits auxiliaires.

10. Système selon la revendication 6, dans lequel le critère de convergence est satisfait lorsqu'une norme d'une différence entre la matrice de densité d'une itération courante et la matrice de densité d'une itération précédente est inférieure à une valeur de tolérance prédéfinie.

11. Un ou plusieurs supports de stockage d'informations lisibles par machine non transitoires comprenant une ou plusieurs instructions qui, lorsqu'elles sont exécutées par un ou plusieurs processeurs matériels classiques et une pluralité de processeurs quantiques, QPU, non enchevêtrées, dans lequel les un ou plusieurs processeurs matériels classiques sont couplés de manière communicante à la pluralité de QPU non enchevêtrées par des interfaces de communication, amènent :

la réception, via les un ou plusieurs processeurs matériels classiques, d'un composé chimique dont une ou plusieurs propriétés doivent être extraites, dans lequel le composé chimique est l'un parmi (i) une molécule, et (ii) un solide ;

l'obtention, via les un ou plusieurs processeurs matériels classiques, de coordonnées atomiques de chacun d'une pluralité d'atomes présents dans le composé chimique ;

la détermination, via les un ou plusieurs processeurs matériels classiques, d'une pluralité d'intégrales d'électrons, d'une matrice hamiltonienne de coeur, et d'une matrice de colocation à partir des coordonnées atomiques de chacun de la pluralité d'atomes, dans lequel la matrice de colocation est une matrice rectangulaire de dimension Ng et NaO, et dans lequel Ng représente un nombre de points sur une grille numérique, et NaO représente un nombre de fonctions de base ;

la détermination, via la pluralité de QPU non enchevêtrées, d'une matrice de densité du composé chimique à partir de la matrice hamiltonienne de coeur dans lequel la matrice de densité est construite en utilisant (i) une matrice de rotation unitaire de particules uniques, obtenue en diagonalisant la matrice hamiltonienne de coeur, et (ii) des occupations d'électrons ; et

la mise à jour itérative, via la pluralité de QPU non enchevêtrées, de la matrice de densité en calculant une matrice de Fock jusqu'à ce qu'un critère de convergence soit satisfait, dans lequel la mise à jour itérative de la matrice de densité à chaque itération comprend :

le calcul d'une matrice directe, J, à partir de la matrice de densité sur la base d'un sous-ensemble d'intégrales d'électrons ;

la détermination d'une matrice d'échange de corrélation à partir de la matrice de colocation pour une approximation de gradient généralisée par,

le codage d'un gradient de la matrice de colocation en traitant un ensemble de matrices de colocation, dans lequel l'ensemble de matrices de colocation sont codées pour un ensemble de positions (x+/-delta,y,z), (x,y+/-delta,z), (x,y,z+/-delta) pour le nombre de fonctions de base à toutes les positions (x,y,z) en utilisant un premier composant de circuit quantique, un deuxième composant de circuit quantique, un troisième composant de circuit quantique sur (a) des qubits de commande composés d'un premier ensemble de qubits et d'un deuxième ensemble de qubits, et (b) une pluralité de qubits auxiliaires dans lequel le premier ensemble de qubits est associé au nombre de points sur la grille numérique, le deuxième ensemble de qubits est associé au nombre de fonctions de base, et la pluralité de qubits auxiliaires sont utilisés pour stocker des entrées numériques pour l'ensemble de matrices de colocation ;

le codage d'une densité électronique en effectuant une multiplication matricielle séquentielle de la matrice de colocation, la matrice de densité, et la matrice de colocation ;

le calcul d'un gradient de densité électronique à partir de la matrice de colocation, de la matrice de densité, et du gradient de la matrice de colocation en composant un quatrième composant de circuit quantique et un cinquième composant de circuit quantique en utilisant au moins un qubit auxiliaire parmi la pluralité de qubits auxiliaires ;

le calcul d'une dérivée d'une densité d'énergie électronique par rapport à la densité électronique en effectuant une séquence de traitement de signal quantique sur un sixième circuit quantique, dans lequel le sixième composant de circuit quantique code la densité électronique et le gradient de densité électronique ;

le codage d'une matrice Z en composant le sixième composant de circuit quantique et la matrice de colocation ; et

le codage d'une matrice d'échange de corrélation en composant la matrice Z avec la matrice de colocation ;

la détermination de la matrice de Fock en combinant la matrice hamiltonienne de coeur, la matrice de densité, et la matrice d'échange de corrélation ; et

la mise à jour de la matrice de densité en diagonalisant la matrice de Fock ; et

l'utilisation, via les un ou plusieurs processeurs matériels classiques, de la matrice de densité mise à jour du composé chimique, pour extraire les une ou plusieurs propriétés du composé chimique.

12. Un ou plusieurs supports de stockage d'informations lisibles par machine non transitoires selon la revendication 11, dans lequel le codage du gradient de la matrice de colocation comprend :

le codage, via la pluralité de QPU non entremêlées, d'un premier sous-ensemble de matrices de colocation pour un premier ensemble de points de grille ((+delta x, 0,0) et (-delta x, 0,0)) pour obtenir un composant X du gradient de la matrice de colocation en utilisant un premier composant de circuit quantique ;

le codage, via la pluralité de QPU non entremêlées, d'un deuxième sous-ensemble de matrices de colocation pour un deuxième ensemble de points de grille ((0, +delta y, 0) et (0, -delta y, 0)) pour obtenir un composant Y du gradient de la matrice de colocation en utilisant un deuxième composant de circuit quantique ;

le codage, via la pluralité de QPU non entremêlées, d'un troisième sous-ensemble de matrices de colocation pour un ensemble de points de grille ((0, 0, +delta z) et (0, 0, - delta z)) pour obtenir un composant Z du gradient de la matrice de colocation en utilisant un troisième composant de circuit quantique ; et

la composition, via la pluralité de QPU non entremêlées, du premier composant de circuit quantique, du deuxième composant de circuit quantique, et du troisième composant de circuit quantique pour obtenir le gradient de la matrice de colocation.

13. Un ou plusieurs supports de stockage d'informations lisibles par machine non transitoires selon la revendication 11, dans lesquels,

le quatrième composant de circuit quantique code séquentiellement la matrice de colocation, la matrice de densité, et le gradient de la matrice de colocation, dans lequel la matrice de densité est codée sur des qubits de commande composés du deuxième ensemble de qubits et d'au moins un qubit auxiliaire parmi la pluralité de qubits auxiliaires, et

le cinquième composant de circuit quantique code séquentiellement le gradient de la matrice de colocation, la matrice de densité, et la matrice de colocation.

14. Un ou plusieurs supports de stockage d'informations lisibles par machine non transitoires selon la revendication 11, dans lesquels, les (i) qubits de commande composés du premier ensemble de qubits et du deuxième ensemble de qubits, et (ii) de la pluralité de qubits auxiliaires, sont représentés mathématiquement comme :

$$\log Ng + \log Nao + 1 + 2 \text{ qubits}$$

dans lequel $\log Ng$ représente le premier ensemble de qubits, $\log Nao$ représente le deuxième ensemble de qubits, et 1+2 qubits représentent la pluralité de qubits auxiliaires.

15. Un ou plusieurs supports de stockage d'informations lisibles par machine non transitoires selon la revendication 11, dans lesquels le critère de convergence est satisfait lorsqu'une norme d'une différence entre la matrice de densité d'une itération courante et la matrice de densité d'une itération précédente est inférieure à une valeur de tolérance prédéfinie.

100

102

112

MEMORY 110

REPOSITORY 114

I/O INTERFACE
116

CLASSICAL
HARDWARE
PROCESSORS
108

106

104

CONTROL SYSTEM
118

SIGNAL DELIVERY
SYSTEM 120

QUANTUM
PROCESSING UNITS
122

QUANTUM
MEMORY 124

FIG. 1

200

Receiving a chemical compound which is to be designed
with desired properties — 202

↓

Obtaining atomic coordinates of each of a plurality of atoms
present in the chemical compound — 204

↓

Determining a plurality of electron integrals, a core
Hamiltonian matrix, and a collocation matrix from the
atomic coordinates of each of the plurality of atoms — 206

↓

Determining a density matrix of the chemical compound
from the core Hamiltonian matrix — 208

↓

( A )

FIG. 2A

200

(A)

Iteratively updating, via the plurality of unentangled QPUs, the density matrix by computing a Fock matrix until a convergence criteria is met, wherein iteratively updating the density matrix at each iteration comprises : —210

Constructing a direct (J) matrix from the density matrix based on a subset of electron integrals —210a

Determining a correlation exchange matrix from the collocation matrix for generalized gradient approximation by —210b

Encoding a gradient of the collocation matrix by processing a set of collocation matrices —210b1

Encoding an electronic density —210b2

Computing an electronic density gradient —210b3

Computing a derivative of an electronic energy density —210b4

Encoding a Z-matrix —210b5

Encoding a correlation exchange matrix —210b6

(B)

FIG. 2B

200

B

Determining the Fock matrix by combining the core Hamiltonian matrix, the density matrix, and the correlation exchange matrix — 210c

Updating the density matrix by diagonalizing the Fock matrix — 210d

Utilizing the updated density matrix of the chemical compound, to design the chemical compound with desired properties — 212

FIG. 2C

Input: atomic coordinates of each atom in a chemical compound whose desired properties must be extracted

Determine electron integrals, a core Hamiltonian, and a collocation matrix

Compute density matrix (DM) by diagonalizing the core Hamiltonian

Compute direct matrix based on DM

Determine a correlation exchange matrix from the collocation matrix for generalized gradient approximation

DM = updated DM

Compute a Fock matrix by adding the direct matrix and the correlation exchange matrix

Perform qubitized diagonalization of the Fock matrix to obtain updated DM

Is convergence criteria satisfied?

No

Yes

Final DM = updated DM

Extract desired properties of the chemical compound using final DM

FIG. 3

FIG. 4

**EP 4 575 929 B1**

FIG. 5

38

FIG. 6

FIG. 7

FIG. 8

FIG. 9

FIG. 10

FIG. 11

FIG. 12

FIG. 13

**EP 4 575 929 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20220012382 A1

- WO 202321061415 A

**Non-patent literature cited in the description**

- **ROSSMANNEK MAX et al.** *Quantum HF/DFT-embedding algorithms for electronic structure calculations: Scaling up to complex molecular systems*
- **TAEHEE et al.** *Implementation of the Density-functional Theory on Quantum Computers with Linear Scaling with respect to the Number of Atoms*
- **ROBERT SCHADE et al.** *Parallel Quantum Chemistry on Noisy Intermediate Scale Quantum Computers*

- **ROSSMANNEK MAX et al.** *Quantum Embedding Method for the Simulation of Strongly Correlated Systems on Quantum Computers*
- **SAMBIT DAS et al.** DFT-FE 1.0: A massively parallel hybrid CPU-GPU density functional theory code using finite-element discretization. *Comput. Phys. Commun.*, 2020, vol. 246, 106853
- **RYAN PEDERSON et al.** *Large scale quantum chemistry with Tensor Processing Units*